(19) Europäisches Patentamt
European Patent Office
Office européen des brevets

(11) EP 2 155 153 B1

(12) **EUROPEAN PATENT SPECIFICATION**

(45) Date of publication and mention of the grant of the patent:
**01.03.2017 Bulletin 2017/09**

(21) Application number: **08759454.5**

(22) Date of filing: **07.05.2008**

(51) Int Cl.:
***A61K 8/34*** (2006.01)
***A61K 8/36*** (2006.01)
***A61K 8/06*** (2006.01)
***A61K 8/39*** (2006.01)
***A61Q 5/10*** (2006.01)
***A61K 8/49*** (2006.01)
***A61K 8/60*** (2006.01)

(86) International application number:
**PCT/EP2008/055652**

(87) International publication number:
**WO 2008/138844 (20.11.2008 Gazette 2008/47)**

(54) **DYEING COMPOSITION COMPRISING A SPECIFIC AMINOPYRAZOLOPYRIDINE OXIDATION BASE, A COUPLER AND A SPECIFIC SURFACTANT**

FÄRBEMITTEL ENTHALTEND EIN SPEZIFISCHES AMINOPYRAZOLOPYRIDINE ALS ENTWICKLER, EINEN KUPPLER UND EIN SPEZIELLES TENSID

COMPOSITION COLORANTE COMPRENANT UNE BASE D'OXYDATION AMINOPYRAZOLOPYRIDINE, UN COUPLEUR ET UN TENSIOACTIF SPÉCIFIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.05.2007 FR 0754954**
**01.06.2007 US 924853 P**

(43) Date of publication of application:
**24.02.2010 Bulletin 2010/08**

(73) Proprietor: **L'Oréal**
**75008 Paris (FR)**

(72) Inventor: **SAUNIER, Jean-Baptiste**
**F-75014 Paris (FR)**

(74) Representative: **Fevrier, Murielle Françoise E.**
**L'Oréal**
**Service DIPI**
**9 Rue Pierre Dreyfus**
**92110 Clichy (FR)**

(56) References cited:
**EP-A- 1 792 606**
**EP-A- 1 792 903**
**WO-A-01/35917**
**WO-A-02/076416**
**WO-A-02/076417**
**WO-A-02/076419**
**WO-A-03/028688**
**US-A1- 2006 277 692**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 155 153 B1

## Description

A subject-matter of the invention is a dyeing composition comprising at least one specific aminopyrazolopyridine base, at least one coupler and at least one specific surfactant. Another subject-matter of the invention is the use of this composition for the dyeing of keratinous fibres and the dyeing method employing this composition.

It is known to dye keratinous fibres, in particular human hair, with dyeing compositions comprising oxidation dye precursors, generally known as oxidation bases, such as ortho- or para-phenylenediamines, ortho- or para-aminophenols and heterocyclic compounds. These oxidation bases are colourless or weakly coloured compounds which, in combination with oxidizing products, can give rise, by an oxidative coupling process, to coloured compounds.

It is also known that it is possible to vary the shades obtained with these oxidation bases by combining them with couplers or colouring modifiers, the latter being chosen in particular from aromatic meta-diamines, meta-aminophenols, meta-diphenols and certain heterocyclic compounds, such as indole compounds.

The variety of the molecules employed as oxidation bases and couplers makes it possible to obtain a rich palette of colours.

The "permanent" colouring obtained by virtue of these oxidation dyes furthermore has to satisfy a certain number of requirements. Thus, it must be without disadvantage toxicologically, it must make it possible to obtain shades within the desired intensity and it must behave well in the face of external agents, such as light, bad weather, washing, permanent waving, perspiration and rubbing.

The dyes must also make it possible to cover white hair and, finally, be as non-selective as possible, that is to say make it possible to obtain the smallest possible differences in colouring along the same keratinous fibre, which is generally differentially sensitized (i.e. damaged) between its tip and its root.

It is already known to use aminopyrazolopyridine oxidation bases for the dyeing of keratinous fibres, in particular in Patent Application FR 2 801 308. These bases make it possible to obtain varied shades.

However, it is desirable to further improve the power of the colouring obtained, its selectivity and its resistance to external agents.

This aim is achieved with the present invention, a subject-matter of which is a dyeing composition as defined in claim 1

Another subject-matter of the invention is a dyeing method employing this composition.

Another subject-matter of the invention is the use of the composition of the present invention for the dyeing of keratinous fibres, in particular human keratinous fibres, such as the hair.

The composition of the present invention makes it possible in particular to obtain a composition for the dyeing of keratinous fibres which is suitable for use in oxidation dyeing and which makes it possible to obtain a colouring with varied, powerful, attractive and not very selective shades which is highly resistant to the various attacks to which hair may be subjected, such as shampoos, light, sweat and permanent deformations.

It should be noted that, in that which follows and unless otherwise indicated, the limits of a range of values are included within this range.

In the compounds of formula (I') above, the expression "alkyl" used for the alkyl radicals and for the groups comprising an alkyl part means a linear or branched carbon chain comprising from 1 to 4 carbon atoms which is unsubstituted or substituted by one or more heterocycles or by one or more phenyl groups or by one or more groups chosen from halogen atoms, such as chlorine, bromine, iodine and fluorine, or hydroxyl, alkoxy, amino, carbonyl, carboxamido, sulphonyl, $-CO_2H$, $-SO_3H$, $-PO_3H_2$, $-PO_4H_2$, $-NHSO_3H$, sulphonamido, mono ($C_1$-$C_4$) alkylamino or tri ($C_1$-$C_4$)alkylammonium radicals, or else by a di($C_1$-$C_4$)alkylamino radical in which the two alkyl groups can form, together with the nitrogen atom of the said di($C_1$-$C_4$)alkylamino group to which they are bonded, a ring which can be interrupted by one or more nitrogen, oxygen or sulphur atoms.

Likewise, according to the invention, the expression "alkoxy" used for alkoxy radicals and for the groups comprising an alkoxy part means a linear or branched O-carbon chain comprising from 1 to 4 carbon atoms which is unsubstituted or substituted by one or more groups chosen from heterocycles; halogen atoms, such as chlorine, bromine, iodine and fluorine; or hydroxyl, amino, carbonyl, carboxamido, sulphonyl, $-CO_2H$, $-SO_3H$, $-PO_3H_2$, $-PO_4H_2$, $-NHSO_3H$, sulphonamido, mono ($C_1$-$C_4$)alkylamino or tri($C_1$-$C_4$)alkylammonium radicals, or else by a di($C_1$-$C_4$)alkylamino radical in which the two alkyl groups can form, together with the nitrogen atom of the said di($C_1$-$C_4$)alkylamino group to which they are bonded, a ring which can be interrupted by one or more nitrogen, oxygen or sulphur atoms.

According to the invention, the term "heterocycle" is understood to mean a 5-, 6-, 7- or 8-membered aromatic or non-aromatic ring comprising from 1 to 3 heteroatoms chosen from nitrogen, sulphur and oxygen atoms. These heterocycles can be fused to other heterocycles or to a phenyl group. They can be substituted by a halogen atom; a ($C_1$-$C_4$)alkyl radical; a ($C_1$-$C_4$)alkoxy radical; a hydroxyl radical; an amino radical; a ($C_1$-$C_4$)alkylamino radical; or a di($C_1$-$C_4$)alkylamino radical in which the two alkyl groups can, together with the nitrogen atom to which they are bonded, form a ring which can be interrupted by one or more nitrogen, oxygen or sulphur atoms. In addition, these heterocycles can be quaternized by a ($C_1$-$C_4$)alkyl radical.

Mention may in particular be made, among these optionally fused heterocycles, by way of example, of the following rings: thiadiazole, triazole, isoxazole, oxazole, azaphosphole, thiazole, isothiazole, imidazole, pyrazole, triazine, thiazine, pyrazine, pyridazine, pyrimidine, pyridine, diazepine, oxazepine, benzotriazole, benzoxazole, benzimidazole, benzothiazole, morpholine, piperidine, piperazine, azetidine, pyrrolidine, aziridine, 3-(2-hydroxyethyl)benzothiazol-3-ium and 1-(2-hydroxyethyl)pyridinium.

According to the invention, the term "phenyl" is understood to mean a phenyl radical which is unsubstituted or substituted by one or more cyano, carbonyl, carboxamido, sulphonyl, $-CO_2H$, $-SO_3H$, $-PO_3H_2$, $-PO_4H_2$, hydroxyl, amino, mono($C_1$-$C_4$)alkylamino or di($C_1$-$C_4$)alkylamino radicals, it being possible, in the case of the last radical, for the two alkyl groups to form, together with the nitrogen atom of the said di($C_1$-$C_4$)alkylamino group to which they are bonded, a ring which can be interrupted by one or more nitrogen, oxygen or sulphur atoms.

The term "cationic ring" or "cationic heterocycle" is understood to mean a ring comprising one or more quaternary ammonium groups.

Mention may be made, as examples of radicals of the $-N^+R_{17}R_{18}R_{19}$ type, of the trimethylammonium, triethylammonium, dimethylethylammonium, diethylmethylammonium, diisopropylmethylammonium, diethylpropylammonium, (β-hydroxyethyl)diethylammonium, di(β-hydroxyethyl)methylammonium or tri(β-hydroxyethyl)ammonium radicals.

Mention may be made, as example of cationic heterocycle, of imidazolium, pyridinium, piperazinium, pyrrolidinium, morpholinium, pyrimidinium, thiazolium, benzimidazolium, benzothiazolium, oxazolium, benzotriazolium, pyrazolium, triazolium or benzoxazolium heterocycles.

The compounds of formula (I') can optionally be salified with strong inorganic acids, such as, for example, HCl, HBr, HI, $H_2SO_4$ or $H_3PO_4$, or organic acids, such as, for example, acetic acid, lactic acid, tartaric acid, citric acid, succinic acid, benzenesulphonic acid, paratoluenesulphonic acid, formic acid or methanesulphonic acid.

If they have anionic groups, such as $-CO_2H$, $-SO_3H$, $-PO_3H_2$ or $-PO_4H_2$ groups, the compounds of formula (I) can be salified by alkali metal or alkaline earth metal hydroxides, such as sodium hydroxide or potassium hydroxide, by aqueous ammonia or by organic amines.

They can also be in the form of solvates, for example a hydrate or a solvate of a linear or branched alcohol, such as ethanol or isopropanol.

In the context of the invention, the term "derivative of formula (I')" is understood to mean all mesomeric or isomeric forms.

Mention may be made, as examples of derivatives of formula (I), of the following compounds, in which $X^-$ is as defined above:

| |
|---|
| 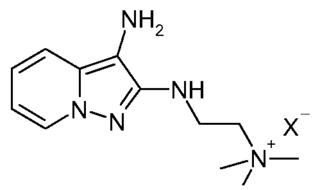 [2-(3-Aminopyrazolo[1,5-a]pyridin-2-ylamino)ethyl]trimethylammonium salt |
| 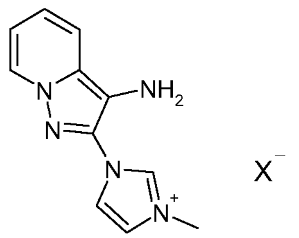 3-(3-Aminopyrazolo[1,5-a]pyridin-2-yl)-1-methyl-3H-imidazol-1-ium salt |
| 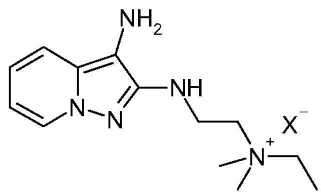 [2-(3-Aminopyrazolo[1,5-a]pyridin-2-ylamino)ethyl]ethyldimethylammonium salt |

(continued)

| |
|---|
| 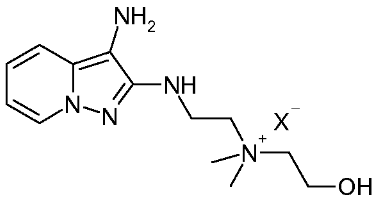 |
| [2-(3-Aminopyrazolo[1,5-a]pyridin-2-ylamino)ethyl](2-hydroxyethyl)dimethylammonium salt |
| 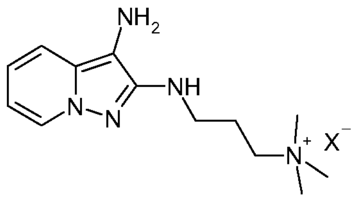 |
| [3-(3-Aminopyrazolo[1,5-a]pyridin-2-ylamino)propyl]trimethylammonium salt |
| 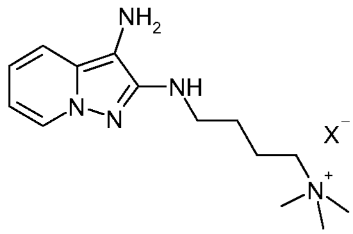 |
| [4-(3-Aminopyrazolo[1,5-a]pyridin-2-ylamino)butyl]-trimethylammonium salt |
| 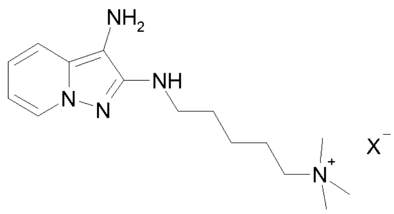 |
| [5-(3-Aminopyrazolo[1,5-a]pyridin-2-ylamino)pentyl]trimethylammonium salt |
| 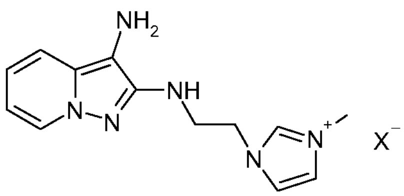 |
| 3-[2-(3-Aminopyrazolo[1,5-a]pyridin-2-ylamino)ethyl]-1-methyl-3H-imidazol-1-ium salt |
| 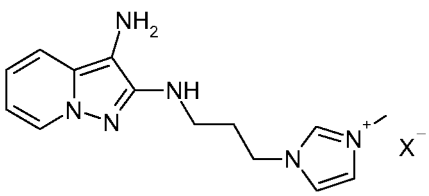 |
| 3-[3-(3-Aminopyrazolo[1,5-a]pyridin-2-ylamino)propyl]-1-methyl-3H-imidazol-1-ium salt |

(continued)

| |
|---|
| 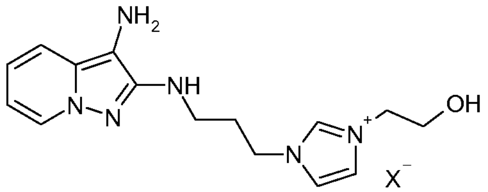<br>3-[3-(3-Aminopyrazolo[1,5-a]pyridin-2-ylamino)propyl]-1-(2-hydroxyethyl)-3H-imidazol-1-ium salt |
| 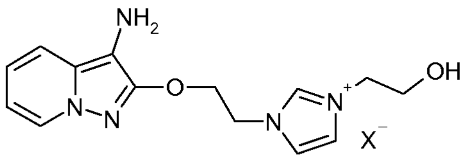<br>3-[2-(3-Aminopyrazolo[1,5-a]pyridin-2-yloxy)ethyl]-1-(2-hydroxyethyl)-3H-imidazol-1-ium salt |
| 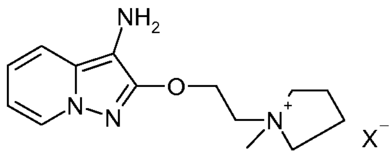<br>1-{2-[(3-Aminopyrazolo[1,5-a]pyridin-2-yl)oxy]ethyl}-1-methylpyrrolidinium salt |
| 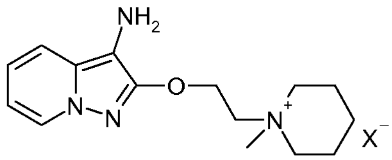<br>1-{2-[(3-Aminopyrazolo[1,5-a]pyridin-2-yl)oxy]ethyl}-1-methylpiperidinium salt |
| 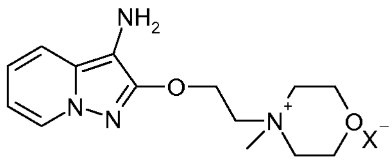<br>4-{2-[(3-Aminopyrazolo[1,5-a]pyridin-2-yl)oxy]ethyl}-4-methylmorpholin-4-ium salt |
| 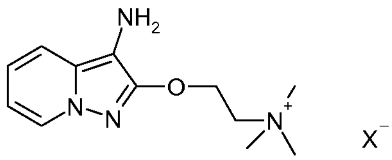<br>{2-[(3-Aminopyrazolo[1,5-a]pyridin-2-yl)oxy]ethyl}trimethylammonium salt |
| 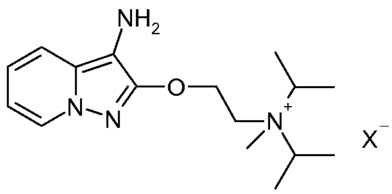<br>{2-[(3-Aminopyrazolo[1,5-a]pyridin-2-yl)oxy]ethyl}diisopropylmethylammonium salt |
| 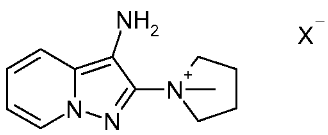 |

(continued)

| |
|---|
| 1-(3-Aminopyrazolo[1,5-a]pyridin-2-yl)-1-methylpyrrolidinium salt |
| 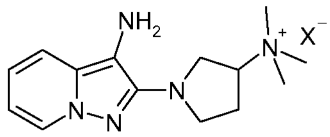<br>[1-(3-Aminopyrazolo[1,5-a]pyridin-2-yl)pyrrolidin-3-yl]trimethylammonium salt |
| 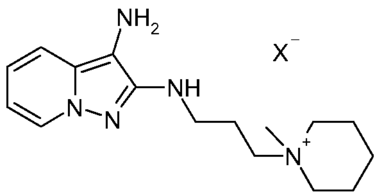<br>1-[3-(3-Aminopyrazolo[1,5-a]pyridin-2-ylamino)propyl]-1-methylpiperidinium salt |
| 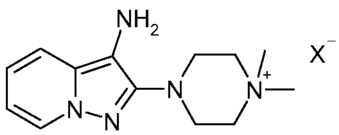<br>4-(3-Aminopyrazolo[1,5-a]pyridin-2-yl)-1,1-dimethylpiperazin-1-ium salt |
| 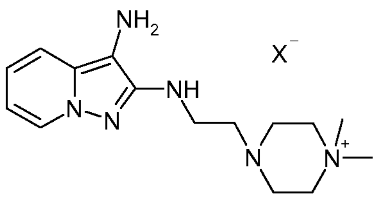<br>4-[2-(3-Aminopyrazolo[1,5-a]pyridin-2-ylamino)ethyl]-1,1-dimethylpiperazin-1-ium salt |
| 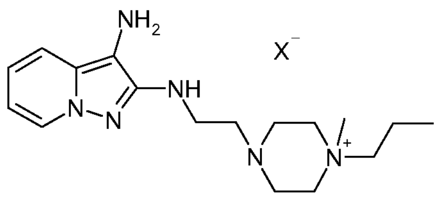<br>4-[2-(3-Aminopyrazolo[1,5-a]pyridin-2-ylamino)ethyl]-1-methyl-1-propylpiperazin-1-ium salt |
| 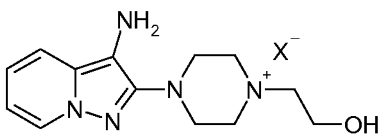<br>4-(3-Aminopyrazolo[1,5-a]pyridin-2-yl)-1-(2-hydroxyethyl)piperazin-1-ium salt |
| 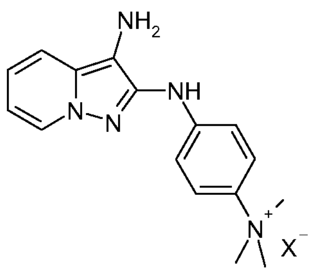<br>[4-(3-Aminopyrazolo[1,5-a]pyridin-2-ylamino)phenyl]trimethylammonium salt |

(continued)

| |
|---|
| 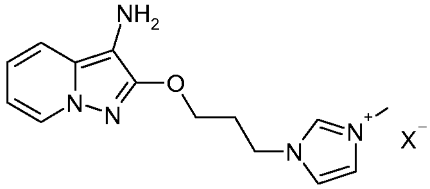<br>3-[3-(3-Aminopyrazolo[1,5-a]pyridin-2-yloxy)propyl]-1-methyl-3H-imidazol-1-ium salt |
| 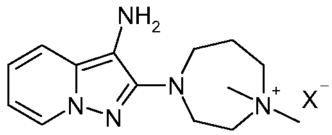<br>4-(3-Aminopyrazolo[1,5-a]pyridin-2-yl)-1,1-dimethyl-1,4-diazepan-1-ium salt |
| 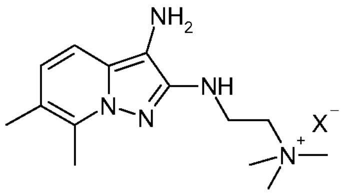<br>[2-(3-Amino-6,7-dimethylpyrazolo[1,5-a]pyridin-2-ylamino)ethyl]trimethylammonium salt |
| 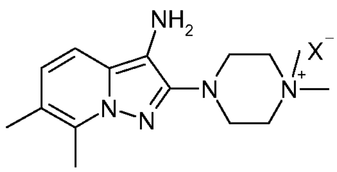<br>4-(3-Amino-6,7-dimethylpyrazolo[1,5-a]pyridin-2-yl)-1,1-dimethylpiperazin-1-ium salt |
| 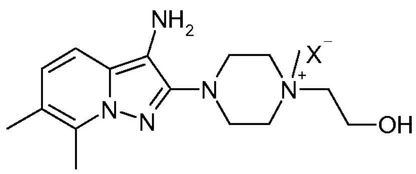<br>4-(3-Amino-6,7-dimethylpyrazolo[1,5-a]pyridin-2-yl)-1-(2-hydroxyethyl)-1-methylpiperazin-1-ium salt |
| 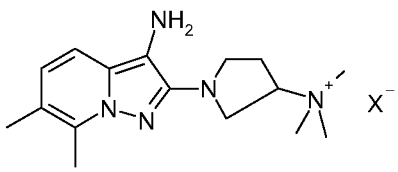<br>[1-(3-Amino-6,7-dimethylpyrazolo[1,5-a]pyridin-2-yl)pyrrolidin-3-yl]trimethylammonium salt |
| 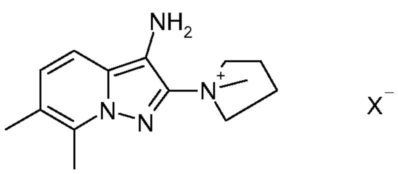<br>1-(3-Amino-6,7-dimethylpyrazolo[1,5-a]pyridin-2-yl)-1-methylpyrrolidinium salt |

(continued)

| |
|---|
| 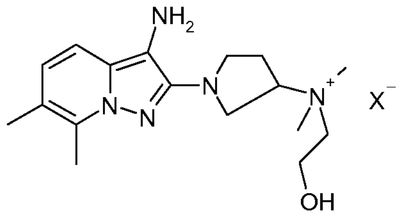 [1-(3-Amino-6,7-dimethylpyrazolo[1,5-a]pyridin-2-yl)pyrrolidin-3-yl](2-hydroxyethyl)dimethylammonium salt |
| 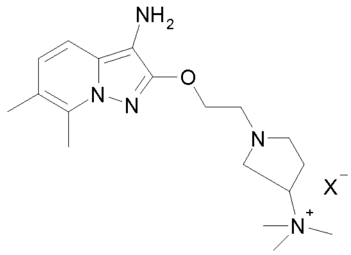 {1-[2-(3-Amino-6,7-dimethylpyrazolo[1,5-a]pyridin-2-yloxy)ethyl]pyrrolidin-3-yl}trimethylammonium salt |
| 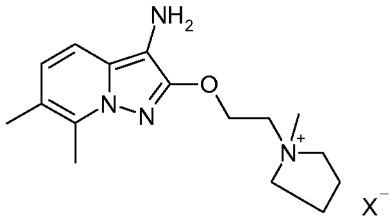 1-{2-[(3-Amino-6,7-dimethylpyrazolo[1,5-a]pyridin-2-yl)oxy]ethyl}-1-methylpyrrolidinium salt |
| 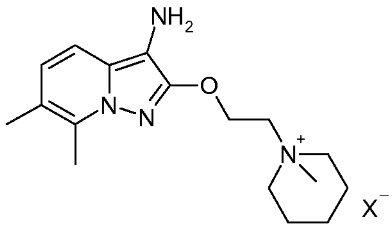 1-{2-[(3-Amino-6,7-dimethylpyrazolo[1,5-a]pyridin-2-yl)oxy]ethyl}-1-methylpiperidinium salt |
| 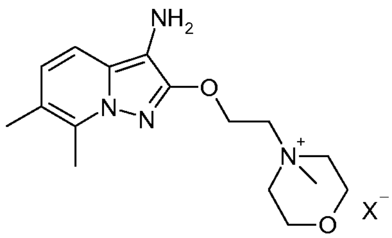 4-{2-[(3-Amino-6,7-dimethylpyrazolo[1,5-a]pyridin-2-yl)oxy]ethyl}-4-methylmorpholin-4-ium salt |
| 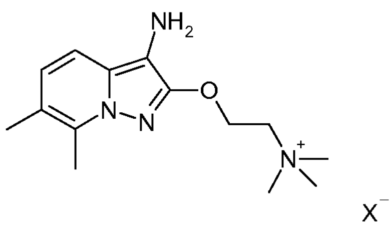 {2-[(3-Amino-6,7-dimethylpyrazolo[1,5-a]pyridin-2-yl)oxy]ethyl}trimethylammonium salt |

(continued)

| |
|---|
| 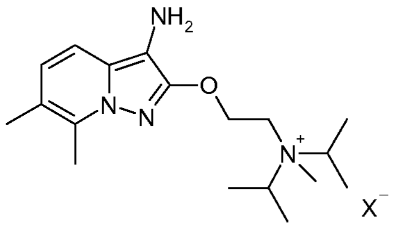<br>{2-[(3-Amino-6,7-dimethylpyrazolo[1,5-a]pyridin-2-yl)oxy]ethyl}diisopropylmethylammonium salt |
| 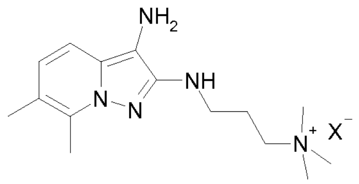<br>[3-(3-Amino-6,7-dimethylpyrazolo[1,5-a]pyridin-2-ylamino)propyl]trimethylammonium salt |
| 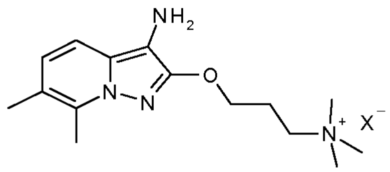<br>[3-(3-Amino-6,7-dimethylpyrazolo[1,5-a]pyridin-2-yloxy)propyl]trimethylammonium salt |
| 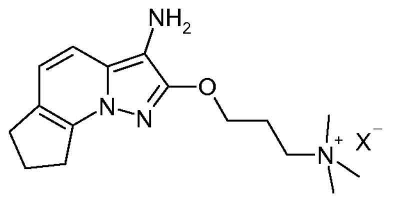<br>[3-(3-Amino-7,8-dihydro-6H-cyclopenta[elpyrazolo[1,5-a]pyridin-2-yloxy)propyl]trimethylammonium salt |

The nature of the counter-ion is not determining with regard to the dyeing power of the compounds of formula (I').

R'$_1$ denotes a heterocycleselected from imidazoles substituted by a quaternary ammonium radical or imidazoliums, piperazines substituted by a quaternary ammonium radical or piperaziniums, pyrrolidines substituted by a quaternary ammonium radical or pyrrolidiniums, or diazepanes substituted by a quaternary ammonium radical or diazepaniums, trialkylammonium, tri(hydroxyalkyl)ammonium, (hydroxyalkyl)dialkylammonium or di(hydroxyalkyl)alkylammonium.

The R'$_3$, R'$_4$ and R'$_5$ radicals can independently be a hydrogen atom or an optionally substituted $C_1$-$C_4$ alkyl radical. Mention may be made, by way of example, of the methyl, ethyl, hydroxyethyl, aminoethyl, propyl or butyl radicals. According to a specific embodiment, R'$_3$, R'$_4$ and R'$_5$ independently represent a hydrogen atom or a $C_1$-$C_4$ alkyl radical.

The oxidation base or bases of the invention are generally each present in an amount of between 0.001 and 10% by weight approximately of the total weight of the dyeing composition, preferably between 0.005 and 6%.

The dyeing composition according to the invention comprises one or more couplers conventionally used for the dyeing of keratinous fibres. Mention may in particular be made, among these couplers, of meta-phenylenediamines, meta-aminophenols, meta-diphenols, naphthalene couplers, heterocyclic couplers and their addition salts.

Mention may be made, as example of coupler, of 2-methyl-5-aminophenol, 5-N-(β-hydroxyethyl)amino-2-methylphenol, 6-chloro-2-methyl-5-aminophenol, 3-aminophenol, 2,4-dichloro-3-aminophenol, 5-amino-4-chloro-o-cresol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-(β-hydroxyethylamino)-3,4-methylenedioxybenzene, α-naphthol, 2-methyl-1-naphthol, 1,5-dihydroxynaphthalene, 2,7-naphthalenediol, 1-acetoxy-2-methylnaphthalene, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 2,6-dihydroxy-3-4-dimethylpyridine, 3-amino-2-methylamino-6-methoxypyridine, 1-N-(β-hydroxyethyl)amino-3,4-methylenedioxybenzene, 2,6-bis(β-hydroxyethylamino)toluene,

3-methyl-1-phenyl-5-pyrazolone, and their addition salts with an acid.

In the composition of the present invention, the coupler or couplers generally represent an amount of between 0.001 and 10% by weight approximately of the total weight of the dyeing composition, preferably between 0.005 and 6%.

The dyeing composition of the invention can optionally comprise one or more additional oxidation bases conventionally used for the dyeing of keratinous fibres other than the oxidation bases of formula (I).

By way of example, these additional oxidation bases can be chosen from para-phenylenediamines other than those described above, bis-phenylalkylenediamines, para-aminophenols, bis-para-aminophenols, ortho-aminophenols, heterocyclic bases other than the oxidation bases of formula (I), and their addition salts.

Mention may be made, among para-phenylenediamines, by way of example, of para-phenylenediamine, para-toluenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine,
4-N,N-bis(β-hydroxyethyl)amino-2-methylaniline,
4-N,N-bis(β-hydroxyethyl)amino-2-chloroaniline,
2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl, β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)-para-phenylenediamine, 4-aminophenylpyrrolidine, 2-thienyl-para-phenylenediamine, 2-(β-hydroxyethylamino)-5-aminotoluene, 3-hydroxy-1-(4'-aminophenyl)pyrrolidine and their addition salts with an acid.

Among the para-phenylenediamines mentioned above, para-phenylenediamine, para-toluenediamine, 2-isopropyl-para-phenylenediamine, 2-(β-hydroxyethyl)-para-phenylenediamine, 2-(β-hydroxyethyloxy)-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine, 2-(β-acetylaminoethyloxy)-para-phenylenediamine and their addition salts with an acid are particularly preferred.

Mention may be made, among bisphenylalkylenediamines, by way of example, of N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane and their addition salts with an acid.

Mention may be made, among para-aminophenols, by way of example, of para-aminophenol, 4-amino-3-methylphenol, 4-amino-3-fluorophenol, 4-amino-3-(hydroxymethyl)phenol, 4-amino-2-methylphenol, 4-amino-2-(hydroxymethyl)phenol, 4-amino-2-(methoxymethyl)phenol, 4-amino-2-(aminomethyl)phenol, 4-amino-2-((β-hydroxyethyl)aminomethyl)phenol, 4-amino-2-fluorophenol, 1-hydroxy-4-(methylamino)benzene, 2,2'-methylenebis(4-aminophenol) and their addition salts with an acid.

Mention may be made, among ortho-aminophenols, by way of example, of 2-aminophenol, 2-amino-5-methylphenol, 2-amino-6-methylphenol, 5-acetamido-2-aminophenol and their addition salts with an acid.

Mention may be made, among heterocyclic bases, by way of example, of pyridine derivatives, pyrimidine derivatives and pyrazole derivatives.

Mention may be made, among pyridine derivatives, of the compounds described, for example, in Patents GB 1 026 978 and GB 1 153 196, such as 2,5-diaminopyridine, 2-[(4-methoxyphenyl)amino]-3-aminopyridine, 2,3-diamino-6-methoxypyridine, 2-[(β-methoxyethyl)amino]-3-amino-6-methoxypyridine, 3,4-diaminopyridine and their addition salts with an acid.

Mention may be made, among pyrimidine derivatives, of the compounds disclosed, for example, in Patents DE 2 359 399; JP 88-169571; JP 05-63124; EP 0 770 375 or Patent Application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine or 2,5,6-triaminopyrimidine, and pyrazolopyrimidine derivatives, such as those mentioned in Patent Application FR-A-2 750 048 and among which may be mentioned pyrazolo[1,5-a]pyrimidine-3,7-diamine; 2,5-dimethylpyrazolo[1,5-a]pyrimidine-3,7-diamine; pyrazolo[1,5-a]pyrimidine-3,5-diamine; 2,7-dimethylpyrazolo[1,5-a]pyrimidine-3,5-diamine; 3-aminopyrazolo[1,5-a]pyrimidin-7-ol; 3-aminopyrazolo[1,5-a]pyrimidine-5-ol; 2-(3-aminopyrazolo[1,5-a]pyrimidin-7-ylamino)ethanol, 2-(7-aminopyrazolo[1,5-a]pyrimidin-3-ylamino)ethanol, 2-[(3-aminopyrazolo[1,5-a]pyrimidin-7-yl)(2-hydroxyethyl)amino]ethanol, 2-[(7-aminopyrazolo[1,5-a]pyrimidin-3-yl)(2-hydroxyethyl)amino]ethanol, 5,6-dimethylpyrazolo[1,5-a]pyrimidine-3,7-diamine, 2,6-dimethylpyrazolo[1,5-a]pyrimidine-3,7-diamine, 2,5,N7,N7-tetramethylpyrazolo[1,5-a]pyrimidine-3,7-diamine, 3-amino-5-methyl-7-(imidazolylpropylamino)pyrazolo[1,5-a]pyrimidine and their addition salts with an acid and their tautomeric forms, when a tautomeric equilibrium exists.

Mention may be made, among pyrazole derivatives, of the compounds disclosed in Patents DE 3 843 892 and

DE 4 133 957 and Patent Applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-(tert-butyl)-1-methylpyrazole, 4,5-diamino-1-(tert-butyl)-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-(hydroxymethyl)pyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-(methylamino)pyrazole, 3,5-diamino-4-(3-hydroxyethyl)amino-1-methylpyrazole and their addition salts with an acid.

Mention may also be made of the diaminopyrazolinones described in Patent Application FR 2 886 137 and in particular 2,3-diamino-6,7-dihydro-1H,5H-pyrazol-1-one and its salts.

Generally, the addition salts of the oxidation bases and couplers which can be used in the context of the invention are chosen in particular from the addition salts with an acid, such as hydrochlorides, hydrobromides, sulphates, citrates, succinates, tartrates, lactates, tosylates, benzenesulphonates, phosphates and acetates, and the addition salts with a base, such as sodium hydroxide, potassium hydroxide, aqueous ammonia, amines or alkanolamines.

The dyeing composition in accordance with the invention can additionally comprise one or more direct dyes which can in particular be chosen from nitrobenzene dyes, azo direct dyes or methine direct dyes. These direct dyes can be nonionic, anionic or cationic in nature.

The composition according to the invention additionally comprises one or more surfactants chosen from $(C_8\text{-}C_{30})$alkyl ether carboxylic acids and their salts, $(C_{12}\text{-}C_{30})$alkyl polyglucosides and mono- or polyglycerolated surface-active agents.

More particularly, the alkyl ether carboxylic acid or acids and their salts are chosen from the compounds of following formula (II):

$$R_8 - A - B - \left[CH_2\right]_{q'} - COOX' \qquad \text{(II)}$$

in which:

- $R_8$ represents a linear or branched $C_8\text{-}C_{22}$ alkyl or alkylene radical or a $(C_8\text{-}C_9)$alkylaryl radical, such as a $(C_8\text{-}C_9)$alkylphenyl radical;
- A represents an oxygen atom or a -CO-, -NH- or -CO-O-group;
- B is composed of a random or block sequence of p' units $-C_3H_6O-$ and of n' units $-C_2H_4O-$;
- n' is an integer ranging from 1 to 30;
- p' is an integer ranging from 0 to 15;
- q' is an integer equal to 0 or 1;
- X' denotes a hydrogen atom or else Na, K, Li, ½ Mg or a monoethanolamine, ammonium or triethanolamine residue.

According to a specific embodiment of the invention, the alkyl ether carboxylic acids and their salts are chosen from those of formula (II) in which $R_8$ denotes a $C_8\text{-}C_{22}$, preferably $C_{10}\text{-}C_{18}$, alkyl radical, A represents an oxygen atom, X' denotes a hydrogen or sodium atom, p' = 0, n' varies from 1 to 20 and preferably from 1 to 10, and q' is equal to 0 or 1.

The commercial products can be composed of a mixture of alkyl ether carboxylic acids or of their salts and, in this case, it is the mean of the values of n' or of p' which are shown. Use may preferably be made, among the commercial products, of the products sold by Chem Y under the names:

- Akypo® NP 70 ($R_8$ = nonylphenyl, A = O, n' = 7, p' = 0, q' = 1, X' = H);
- Akypo® NP 40 ($R_8$ = nonylphenyl, A = O, n' = 4, p' = 0, q' = 1, X' = H);
- Akypo® OP 40 ($R_8$ = octylphenyl, A = O, n' = 4, p' = 0, q' = 1, X' = H);
- Akypo® OP 80 ($R_8$ = octylphenyl, A = O, n' = 8, p' = 0, q' = 1, X' = H) ;
- Akypo® OP 190 ($R_8$ = octylphenyl, A = O, n' = 19, p' = 0, q' = 1, X' = H) ;
- Akypo® RLM 38 ($R_8$ = $(C_{12}\text{-}C_{14})$ alkyl, A = O, mean n' = 3.8, p' = 0, q' = 1, X' = H) ;
- Akypo® RLM 38 NV ($R_8$ = $(C_{12}\text{-}C_{14})$ alkyl, A = O, n' = 4, p' = 0, q' = 1, X' = Na);
- Akypo® RLM 45 ($R_8$ = $(C_{12}\text{-}C_{14})$alkyl, A = O, mean n' = 4.5, p' = 0, q' = 1, X' = H) ;
- Akypo® RLM 45 NV ($R_8$ = $(C_{12}\text{-}C_{14})$alkyl, A = O, mean n' = 4.5, p' = 0, q' = 1, X' = Na);
- Akypo® RLM 100 ($R_8$ = $(C_{12}\text{-}C_{14})$alkyl, A = O, n' = 10, p' = 0, q' = 1, X' = H);

- Akypo® RLM 100 NV ($R_8$ = ($C_{12}$-$C_{14}$)alkyl, A = O, n' = 10, p' = 0, q' = 1, X' = Na);
- Akypo® RLM 130 ($R_8$ = ($C_{12}$-$C_{14}$)alkyl, A = O, n' = 13, of 0, q' = 1, X' = H);
- Akypo® RLM 160 NV ($R_8$ = ($C_{12}$-$C_{14}$)alkyl, A = O, n' = 16, p' = 0, q' = 1, X' = Na);
- Akypo® RS 60 ($R_8$ = ($C_{17}$) alkyl, A = -CO-O-, n' = 6, p' = 0, q' = 1, X' = H);
- Akypo® RCS 60 ($R_8$ = cetyl, A = O / $R_8$ = ($C_{17}$)alkyl, A = -CO-O-; n' = 6, p' = 0, q' = 1, X' = H);
- Akypo® RS 100 ($R_8$-A = stearyl, n' = 10, p' = 0, q' = 1, X' = H);
- Akypo® RO 50 ($R_8$-A = oleyl, n' = 5, p' = 0, q' = 1, X' = H) ;
- Akypo® Soft 70 NV;
- Akypo® Soft 45 NV;
- Akypo® Soft 100 NV;
- Akypo® RLM 45 CA;
- Akypo® RLM 70;
- Akypo® TFC;
- Akypo® FOM 30 ($R_8$ = lauryl, A = NH);
- Akypo® Surfine WLL;

or by Sandoz under the names:

- Sandopan ACA-48 ($R_8$ = cetyl, A = O / ($C_{17}$)alkyl, A = -CO-O-; n' = 24, p' = 0, q' = 1, X' = H);
- Sandopan DTC-Acid ($R_8$ = ($C_{13}$)alkyl, A = O, n' = 6, p' = 0, q' = 1, X' = H);
- Sandopan DTC ($R_8$ = ($C_{13}$)alkyl, A = O, n' = 6, p' = 0, q' = 1, X' = Na);
- Sandopan LS 24 ($R_8$ = ($C_{12}$-$C_{14}$)alkyl, A = O, n' = 12, p' = 0, q' = 1, X' = Na);
- Sandopan JA 36 ($R_8$ = ($C_{13}$)alkyl, A = O, n' = 18, p' = 0, q' = 1, X' = H).

Alkyl polyglycoside or polyglycosides are well known and can more particularly be represented by the following general formula:

$$R_9O\text{-}(R_{10}O)_{t'}\text{-}(G)_v \qquad \text{(III)}$$

in which $R_9$ represents a linear or branched alkyl and/or alkylene radical comprising approximately from 12 to 30 carbon atoms or an alkylphenyl radical, the linear or branched alkyl radical of which comprises from 12 to 30 carbon atoms, $R_{10}$ represents one or more alkylene radicals comprising approximately from 2 to 4 carbon atoms, G represents a sugar unit comprising from 5 to 6 carbon atoms, t' denotes a value ranging from 0 to 10, preferably from 0 to 4, and v denotes a value ranging from 1 to 15.

Alkyl polyglycosides which are preferred according to the present invention are compounds of formula (III) in which $R_9$ more particularly denotes a saturated or unsaturated and linear or branched alkyl radical comprising from 12 to 30 carbon atoms, t' denotes a value ranging from 0 to 3 and more particularly still equal to 0, and G can denote glucose, fructose or galactose, preferably glucose. The degree of polymerization, i.e. the value of v in the formula (III), can range from 1 to 15, preferably from 1 to 4. The mean degree of polymerization is more particularly between 1 and 2 and more preferably still from 1.1 to 1.5.

The glycoside bonds between the sugar units are of 1-6 or 1-4 type and preferably of 1-4 type.

Compounds of formula (III) are represented in particular by the products sold by Cognis under the names Plantaren® (600 CS/U, 1200 and 1300) or Plantacare® (818 and 1200).

The mono- or polyglycerolated surface-active agent or agents preferably comprise, on average, from 1 to 30 glycerol groups, more particularly from 1 to 10 glycerol groups and in particular from 1.5 to 5 glycerol groups.

The monoglycerolated or polyglycerolated surface-active agent or agents are preferably chosen from the compounds with the following formulae: $R_{11}O[CH_2CH(CH_2OH)O]_mH$, $R_{11}O[CH_2CH(OH)CH_2O]_mH$ or $R_{11}O[CH(CH_2OH)CH_2O]_mH$, in which $R_{11}$ represents a saturated or unsaturated and linear or branched hydrocarbon radical comprising from 8 to 40 carbon atoms and preferably from 10 to 30 carbon atoms and m is a number between 1 and 30, preferably between 1 and 10, more particularly from 1.5 to 6.

$R_{11}$ can optionally comprise heteroatoms, such as, for example, oxygen and nitrogen. In particular, $R_{11}$ can optionally comprise one or more hydroxyl and/or ether and/or amide groups.

$R_{11}$ preferably denotes a $C_{10}$-$C_{20}$ alkyl or alkylene radical which is optionally mono- or polyhydroxylated.

Use may be made, for example, of the polyglycerolated (3.5 mol) hydroxylauryl ether sold under the name Chimexane® NF by Chimex.

Preferably, the surface-active agent or agents of use in the context of the present invention are chosen from lauryl ether carboxylic acid, ($C_{12}$/$C_{14}$/$C_{16}$ 68/26/6) alkyl polyglucoside and polyglycerolated cetearyl alcohol comprising r mol of glycerol, r being an integer between 2 and 10, preferably between 2 and 6.

The above surface-active agent or agents are each present in the composition in accordance with the invention in an amount of between 0.01% and 30% by weight, preferably from 0.1% to 15% by weight, of the total weight of the dyeing composition.

The medium appropriate for dyeing of keratinous fibres, in particular human keratinous fibres, such as the hair, also referred to as dyeing vehicle, generally comprises water or a mixture of water and of at least one organic solvent in order to dissolve the compounds which would not be sufficiently soluble in water. Mention may be made, as an organic solvent, for example, of lower $C_1$-$C_4$ alkanols, such as ethanol and isopropanol; polyols and polyol ethers, such as 2-butoxyethanol, propylene glycol, propylene glycol monomethyl ether, diethylene glycol monoethyl ether and diethylene glycol monomethyl ether; and also aromatic alcohols, such as benzyl alcohol or phenoxyethanol, and their mixtures.

The solvents are preferably present in proportions preferably of between 1 and 40% by weight approximately, with respect to the total weight of the dyeing composition, and more preferably still between 5 and 30% by weight approximately.

The dyeing composition in accordance with the invention can also include various adjuvants conventionally used in compositions for dyeing the hair, such as anionic, cationic, amphoteric and nonionic surfactants other than those of the invention, anionic, cationic, nonionic, amphoteric or zwitterionic polymers or their mixtures, inorganic or organic thickening agents and in particular anionic, cationic, nonionic and amphoteric polymeric associative thickeners, antioxidants, penetration agents, sequestering agents, fragrances, buffers, dispersing agents, conditioning agents, such as, for example, volatile or non-volatile and modified or unmodified silicones, film-forming agents, ceramides, preservatives or opacifying agents.

The above adjuvants are generally present in an amount, for each of them, of between 0.01 and 20% by weight, with respect to the weight of the composition.

Of course, a person skilled in the art will take care to choose this or these optional additional compounds so that the advantageous properties intrinsically attached to the oxidation dyeing composition in accordance with the invention are not, or not substantially, detrimentally affected by the envisaged addition or additions.

The pH of the dyeing composition in accordance with the invention is generally between 3 and 12 approximately and preferably between 5 and 11 approximately. It can be adjusted to the desired value using acidifying or basifying agents commonly used in the dyeing of keratinous fibres or else by means of conventional buffering systems.

Mention may be made, among acidifying agents, by way of example, of inorganic or organic acids, such as hydrochloric acid, orthophosphoric acid, sulphuric acid, carboxylic acids, such as acetic acid, tartaric acid, citric acid or lactic acid or sulphonic acids.

Mention may be made, among basifying agents, by way of example, of aqueous ammonia, alkaline carbonates, alkanolamines, such as mono-, di- and triethanolamines and their derivatives, sodium hydroxide, potassium hydroxide and the compounds of following formula (IV):

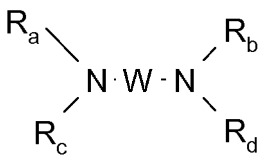

(IV)

in which W is a propylene residue optionally substituted by a hydroxyl group or a $C_1$-$C_4$ alkyl radical and $R_a$, $R_b$, $R_c$ and $R_d$, which are identical or different, represent a hydrogen atom or a $C_1$-$C_4$ alkyl or $C_1$-$C_4$ hydroxyalkyl radical.

The dyeing composition according to the invention can be provided in various forms, such as in the form of liquids, of creams or of gels or in any other form appropriate for carrying out dyeing of keratinous fibres and in particular of human hair.

The method of the present invention is a method in which the composition according to the present invention as defined above is applied to the fibres in the presence of an oxidizing agent for a time sufficient to develop the desired colouring. The colour can be developed at acidic, neutral or alkaline pH and the oxidizing agent can be added to the composition of the invention only at the time of use or it can be employed starting from an oxidizing composition comprising it, applied simultaneously with or sequentially to the composition of the invention.

Development at acidic pH can prove to be particularly advantageous.

According to a specific embodiment, the composition according to the present invention is mixed, preferably at the time of use, with a composition comprising, in a medium appropriate for dyeing, at least one oxidizing agent, this oxidizing agent being present in an amount sufficient to develop a colouring. The mixture obtained is subsequently applied to the keratinous fibres. After a setting time of 3 to 50 minutes approximately, preferably 5 to 30 minutes approximately, the keratinous fibres are rinsed, washed with shampoo, rinsed again and then dried.

The oxidizing agents conventionally used for the oxidation dyeing of keratinous fibres are, for example, hydrogen

peroxide, urea hydrogen peroxide, alkali metal bromates, persalts, such as perborates and persulphates, peracids and oxidase enzymes, among which may be mentioned peroxidases, 2-electron oxidoreductases, such as uricases, and 4-electron oxygenases, such as laccases. Hydrogen peroxide is particularly preferred.

The oxidizing composition can also include various adjuvants conventionally used in compositions for the dyeing of the hair and as defined above.

The pH of the oxidizing composition including the oxidizing agent is such that, after mixing with the dyeing composition, the pH of the resulting composition applied to the keratinous fibres preferably varies between 3 and 12 approximately and more preferably still between 5 and 11. It can be adjusted to the desired value using acidifying or basifying agents commonly used in the dyeing of keratinous fibres and as defined above.

The ready-for-use composition which is finally applied to the keratinous fibres can be provided in various forms, such as in the form of liquids, of creams or of gels, or in any other form appropriate for carrying out dyeing of keratinous fibres and in particular of human hair.

Another subject-matter of the invention is a dyeing kit or multicompartment device in which a first compartment includes the dyeing composition of the present invention defined above and a second compartment includes an oxidizing agent. This device can be equipped with a means allowing the desired mixture to be deposited on the hair, such as the devices described in Patent FR-2 586 913 on behalf of the Applicant Company.

Starting from this device, it is possible to dye keratinous fibres starting from a method which comprises the mixing of a dyeing composition comprising one or more oxidation bases of formula (I), one or more couplers and one or more surfactants according to the invention with an oxidizing agent and the application of the mixture obtained to the keratinous fibres for a time sufficient to develop the desired colouring.

The examples which follow serve to illustrate the invention without, however, exhibiting a limiting nature.

**Examples**

**Composition 1**

Composition 1 comprises the following dyes:

| | |
|---|---|
| 2-[(3-Aminopyrazolo[1,5-a]pyridin-2-yl)amino]-N,N,N-trimethylethanaminium chloride hydrochloride | 0.008 mol% |
| 2-Chloro-6-methyl-3-aminophenol | 0.008 mol% |

introduced into the following medium:

| | |
|---|---|
| ($C_8$/$C_{10}$ 50/50)Alkyl polyglucoside(2) as a 60% aqueous solution | 7.0 g AM* |
| Lauryl alcohol 2 EO (Dehydol®LS-2-DEO-N, sold by Cognis | 4.0 g |
| Decyl alcohol 5 EO (Empilan®KA-5 /90 - FL, sold by Albright & Wilson) | 8.0 g |
| Oleyl alcohol | 3.0 g |
| Monoethanolamide of ($C_{13}$/$C_{15}$) alkyl ether carboxylic acid comprising 2 mol of ethylene oxide | 5 g |
| Cationic associative polymer (Quatrisoft LM 200®, sold by Amerchol) | 1.0 g |
| Monoethanolamine | 2.0 g |
| Polyquaternium 6 (Merquat® 100, sold by Calgon) | 1.5 g |
| Ethanol | 11.0 g |
| Propylene glycol | 5.0 g |
| Dipropylene glycol | 5.0 g |
| Reducing agents, antioxidants | q.s. |
| Sequestering agents | q.s. |
| Fragrance | q.s. |
| Aqueous ammonia (comprising 20.5% of $NH_3$) | 1.6 g |
| Demineralized water q.s. for | 100 g |
| AM* means that the content is shown as grams of active material. | |

**Composition 2**

Composition 2 comprises the following dyes:

| | |
|---|---|
| 2-[(3-Aminopyrazolo[1,5-a]pyridin-2-yl)amino]-N,N,N-trimethylethanaminium chloride hydrochloride | 0.007 mol% |
| meta-Aminophenol | 0.007 mol% |

introduced into the following medium:

| | |
|---|---|
| Mixtures of linear $C_{18}$ to $C_{24}$ [$C_{18}/C_{20}/C_{22}/C_{24}$, 7/58/30/6, contents of alcohols >95%] alcohols (Nafol 20-22) | 3 g |
| Mixtures of linear $C_{18}$ to $C_{24}$ [$C_{18}/C_{20}/C_{22}/C_{24}$ 7/58/30/6, contents of alcohols >95%] alcohols polyglycerolated with 6 mol of glycerol | 1.35 g |
| Cetearyl alcohol polyglycerolated with 2 mol of glycerol | 4.0 g |
| Cetearyl alcohol polyglycerolated with 6 mol of glycerol | 2.0 g |
| Oleic acid | 2.6 g |
| Glycol distearate | 2.0 g |
| Propylene glycol | 7.5 g |
| Monoisopropanolamide of coconut oil acids | 2.0 g |
| Aculyn 44, sold by Röhm & Haas | 1.4 g AM* |
| Crosslinked polyacrylic acid | 0.6 g |
| Cationic polymer of formula (W) | 3 g AM |
| Merquat 100, sold by Calgon | 0.4 g AM |
| Reducing agents | 0.7 g |
| Sequestering agents | 0.2 g |
| Pure monoethanolamine | 1.06 g |
| Aqueous ammonia comprising 20.5% of $NH_3$ | 11.1 g |
| Water | q.s. for 100 g |
| AM* means that the content is shown as grams of active material. | |

**Method of application**

Each composition was mixed at the time of use with 1.5 times its weight of 20-volume aqueous hydrogen peroxide solution, the pH of which is 3.

The mixture thus produced was applied to natural grey hair comprising 90% of white hairs in a proportion of 30 g per 3 g of hair for 30 minutes.

The hair was subsequently rinsed, washed with a standard shampoo and dried.

**Results**

The hair colouring was evaluated visually.

| | height of tone | Highlight |
|---|---|---|
| **Composition 1** | Dark blond | Purplish blue |
| **Composition 2** | Blond | Ash blue |

These shades are long-lasting and uniform.

**Compositions 3 and 4.**

The following compositions were prepared (moles %)

**Composition 3 (comparative) :**

| | |
|---|---|
| 2,3-Diamino-6,7-dihydro-1H,5H-pyrazolo[1,2-a]pyrazol-1-one Dimethanesulfonate (0.005 mol) | 1,732 g |
| 2-Methyl 5-(2-hydroxyethyl) amino phenol (0.005 mol) | 0.835 g |
| **lauryl ether 4,5 OE carboxylic Acid** (Akypo ® RLM 45 sold by CHEM Y) | 7.0 g A.M |
| Lauric alcohol with 2 OE (Dehydol®LS-2-DEO-N sold by COGNIS | 4.0 g |
| Decylic alcohol with 5 OE (Empilan®KA-5 /90 - FL sold by ALBRIGHT & WILSON) | 8.0 g |
| Oleic alcohol | 3.0 g |
| Monoethanolamide of ($C_{13}$/$C_{15}$)alkyl ether carboxylic acid comprising 2 mol of ethylene oxide | 5 g |
| cationic associative polymer (Quatrisoft LM 200® sold by AMERCHOL) | 1.0 g |
| Monoethanolamine | 2 0 g |
| Polyquaternium 6(Merquat ® 100 sold by CALGON) | 1.5 g |
| Ethanol | 11 g |
| Propylene glycol | 5.0 g |
| Dipropylene glycol | 5.0 g |
| Reducing agents, antioxidants | q.s. |
| Sequestering agents | q.s. |
| Fragrance | q.s. |
| Aqueous ammonia (comprising 20.5% of $NH_3$) | 1.6 g |
| Demineralized water q.s. for | 100 g |
| AM* means that the content is shown as grams of active material. | |

**Composition 4 (invention):**

| | |
|---|---|
| 2-[(3-Aminopyrazolo[1,5-a]pyridin-2-yl)oxy]-N,N,N-trimethylethanaminium hydrochloride (0.005 mol) | 1,536 g |
| 2-Methyl 5-(2-hydroxyethyl) amino phenol (0.005 mol) | 0.835 g |
| lauryl ether 4,5 OE carboxylic Acid (Akypo ® RLM 45 sold by CHEM Y) | 7.0 g A.M |
| Lauric alcohol 2 OE (Dehydol®LS-2-DEO-N sold by COGNIS | 4.0 g |
| decylic 5 OE Alcohol (Empilan®KA-5 /90 - FL sold by ALBRIGHT & WILSON) | 8.0 g |
| Oleic alcohol | 3.0 g |
| Monoethanolamide of ($C_{13}$/$C_{15}$)alkyl ether carboxylic acid comprising 2 mol of ethylene oxide | 5 g |
| cationic associative polymer (Quatrisoft LM 200® sold by AMERCHOL) | 1.0 g |
| Monoethanolamine | 2 0 g |
| Polyquaternium 6(Merquat ® 100 sold by CALGON) | 1.5 g |
| Ethanol | 11 g |
| Propylene glycol | 5.0 g |
| Dipropylene glycol | 5.0 g |
| Reducing agents, antioxidants | q.s. |
| Sequestering agents | q.s. |
| Fragrance | q.s. |
| Aqueous ammonia (comprising 20.5% of $NH_3$) | 1.6 g |
| Demineralized water q.s. for | 100 g |
| AM* means that the content is shown as grams of active material. | |

**Method of d'application**

Each composition was mixed at the time of use with 1.5 times its weight of 20-volume aqueous hydrogen peroxide solution, the pH of which is 3.

The mixture thus produced was applied to natural grey hair comprising 90% of white hairs natural or permed

in a proportion of 20 g per 2 g of hair for 30 minutes.

The hair was subsequently rinsed, washed with a standard shampoo and dried.

**Color Determination**

The hair coloration was evaluated in the L*a*b* system, with a spectrophotometer SF600X of Datacolor (diffus, 8°) : opening SAV, mesure in SCI, UVI with illuminant D65.

According to this system, L* indicates the lightness. The lowest is the value of L*, the most intense is the color of the hair. The chromaticity coordinates are expressed by the parameters a* and b*, a* indicating the axis of red/green shades and b* the axis of yellow/blue shades.

**SELECTIVITY OF THE COLORATION**

The selectivity of the coloration is the variation of the color between natural colored hair and permed colored hair. Natural hair is representative of the nature of the hair at the point hair and the permed hair is representative of the nature of the hair at the root.

The selectivity is measured by :

$\Delta E$, which is the color variation between a natural colored lock and a permed colored lock, is obtained from the following formula:

$$\Delta E = \sqrt{(L^* - L_o^*)^2 + (a^* - a_o^*)^2 + (b^* - b_o^*)^2}$$

wherein L* indicates lightness and a* and b* are the chromaticity coordinates of the natural colored locks whereas $L_0^*$ indicates the lightness and $a_0^*$ et $b_0^*$ are the chromaticity of the permed colored locks. The lowest is the value of $\Delta E$, the weakest the selective is the coloration and the best is the color of the hair.

In the following table are reported the L*a*b* values measured on natural and permed hair after coloration, and the selectivity values.

| | *Values after coloration of the natural hair* | | | *Values after coloration on permed hair* | | | *Selectivity* |
|---|---|---|---|---|---|---|---|
| | *L** | *a** | *b** | *L** | *a** | *b** | |
| **Composition 3** | 45.2 | 23.7 | 31.7 | 40. 3 | 31. 7 | 34.7 | **9.8** |
| **Composition 4** | 36.5 | 22.6 | 7.9 | 30. 6 | 26. 0 | 7.6 | **6.8** |

These results show a lower selectivity with composition 4 thant the one obtained from composition 3 comprising a non cationic diaminopyrazolopyridine oxidation base.

## Claims

1. Dyeing composition comprising, in a dyeing medium appropriate for the dyeing of keratinous fibres,

   - one or more aminopyrazolopyridine oxidation bases of following formula (I'):

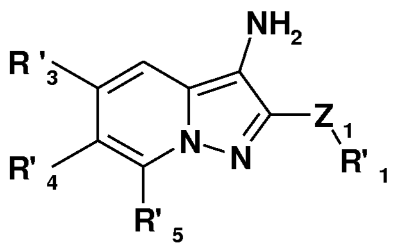

(I')

X

in which:

- $Z_1$ represents:
  - a simple covalent bond,
  - a divalent radical chosen from:
    - an $-O(CH_2)_p-$ radical, p denoting an integer ranging from 0 to 6,
    - an $-NR'_{6}(CH2)_q-$ radical, q denoting an integer ranging from 0 to 6, and $R'_6$ representing a hydrogen atom or a $C_1$-$C_6$ alkyl radical optionally substituted by one or more hydroxyl groups,
- $R'_1$ represents imidazoles substituted by a quaternary ammonium radical or imidazoliums, piperazines substituted by a quaternary ammonium radical or piperaziniums, pyrrolidines substituted by a quaternary ammonium radical or pyrrolidiniums, or diazepanes substituted by a quaternary ammonium radical or diazepaniums ; a trialkylammonium, tri(hydroxyalkyl)ammonium, (hydroxyalkyl)dialkylammonium or di(hydroxyalkyl)alkylammonium group $R'_3$, $R'_4$ and $R'_5$, which are identical or different, represent:
  - a hydrogen atom,
  - an optionally substituted $C_1$-$C_4$ alkyl radical,
- X represents an ion or group of ions which makes it possible to provide the electronegativity of the derivative of formula (I'),

- one or more couplers, and
- one or more surfactants chosen from $(C_8$-$C_{30})$alkyl ether carboxylic acids and their salts, $(C_{12}$-$C_{30})$alkyl polyglucosides and mono- or polyglycerolated surface-active agents.

**2.** Composition according to Claim 1 in which the compound of formula (I) is chosen from:

| |
|---|
| 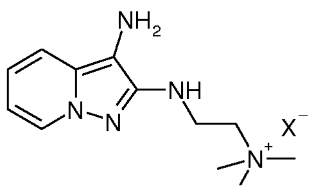<br>[2-(3-Aminopyrazolo[1,5-a]pyridin-2-ylamino)ethyl]trimethylammonium salt |
| 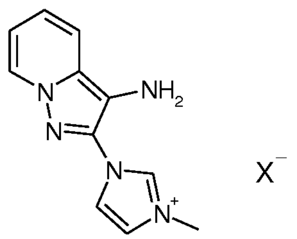<br>3-(3-Aminopyrazolo[1,5-a]pyridin-2-yl)-1-methyl-3H-imidazol-1-ium salt |
| 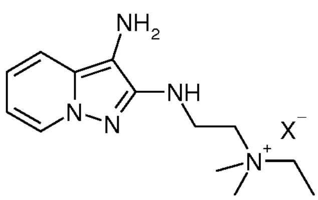<br>[2-(3-Aminopyrazolo[1,5-a]pyridin-2-ylamino)ethyl]ethyldimethylammonium salt |

(continued)

| |
|---|
| 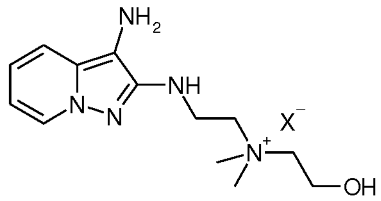 |
| [2-(3-Aminopyrazolo[1,5-a]pyridin-2-ylamino)ethyl](2-hydroxyethyl)dimethylammonium salt |
| 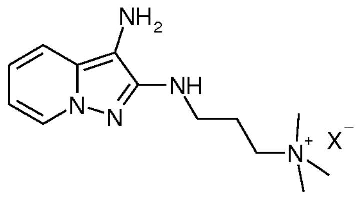 |
| [3-(3-Aminopyrazolo[1,5-a]pyridin-2-ylamino)propyl]trimethylammonium salt |
| 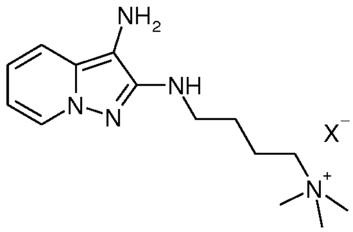 |
| [4-(3-Aminopyrazolo[1,5-a]pyridin-2-ylamino)butyl]-trimethylammonium salt |
| 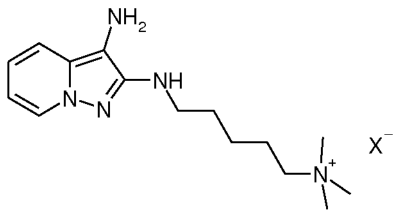 |
| [5-(3-Aminopyrazolo[1,5-a]pyridin-2-ylamino)pentyl]trimethylammonium salt |
| 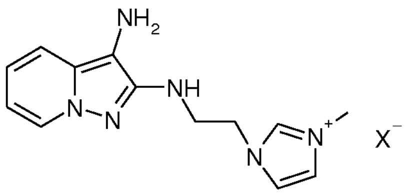 |
| 3-[2-(3-Aminopyrazolo[1,5-a]pyridin-2-ylamino)ethyl]-1-methyl-3H-imidazol-1-ium salt |
| 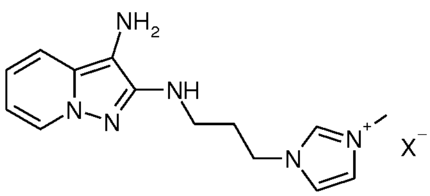 |
| 3-[3-(3-Aminopyrazolo[1,5-a]pyridin-2-ylamino)propyl]-1-methyl-3H-imidazol-1-ium salt |

(continued)

| |
|---|
| 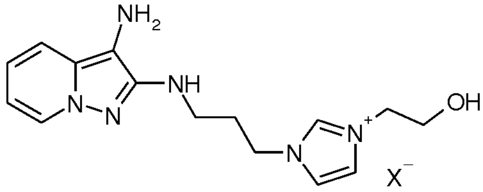<br>3-[3-(3-Aminopyrazolo[1,5-a]pyridin-2-ylamino)propyl]-1-(2-hydroxyethyl)-3H-imidazol-1-ium salt |
| 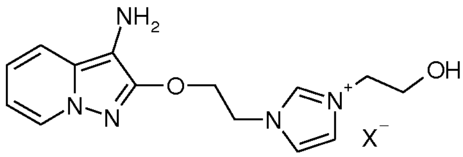<br>3-[2-(3-Aminopyrazolo[1,5-a]pyridin-2-yloxy)ethyl]-1-(2-hydroxyethyl)-3H-imidazol-1-ium salt |
| 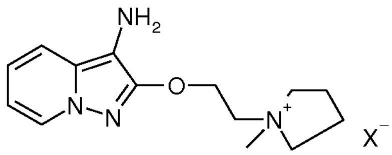<br>1-{2-[(3-Aminopyrazolo[1,5-a]pyridin-2-yl)oxy]ethyl}-1-methylpyrrolidinium salt |
| 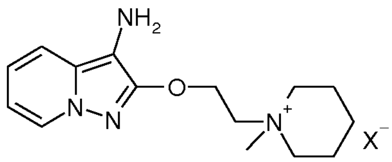<br>1-{2-[(3-Aminopyrazolo[1,5-a]pyridin-2-yl)oxy]ethyl}-1-methylpiperidinium salt |
| 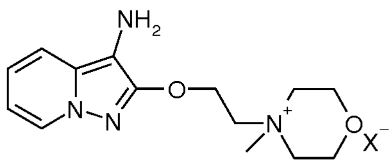<br>4-{2-[(3-Aminopyrazolo[1,5-a]pyridin-2-yl)oxy]ethyl}-4-methylmorpholin-4-ium salt |
| 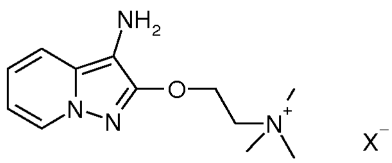<br>{2-[(3-Aminopyrazolo[1,5-a]pyridin-2-yl)oxy]ethyl}trimethylammonium salt |
| 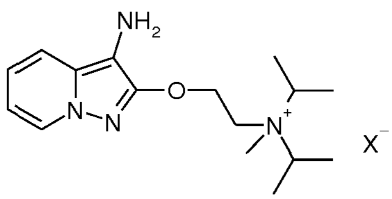<br>{2-[(3-Aminopyrazolo[1,5-a]pyridin-2-yl)oxy]ethyl}diisopropylmethylammonium salt |
| 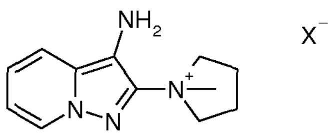 |

(continued)

| |
|---|
| 1-(3-Aminopyrazolo[1,5-a]pyridin-2-yl)-1-methylpyrrolidinium salt |
| 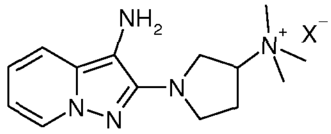 [1-(3-Aminopyrazolo[1,5-a]pyridin-2-yl)pyrrolidin-3-yl]trimethylammonium salt |
| 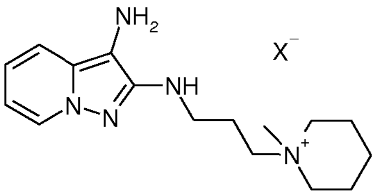 1-[3-(3-Aminopyrazolo[1,5-a]pyridin-2-ylamino)propyl]-1-methylpiperidinium salt |
| 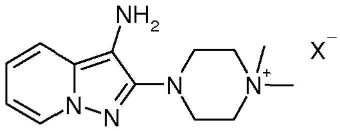 4-(3-Aminopyrazolo[1,5-a]pyridin-2-yl)-1,1-dimethylpiperazin-1-ium salt |
| 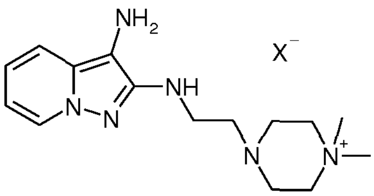 4-[2-(3-Aminopyrazolo[1,5-a]pyridin-2-ylamino)ethyl]-1,1-dimethylpiperazin-1-ium salt |
| 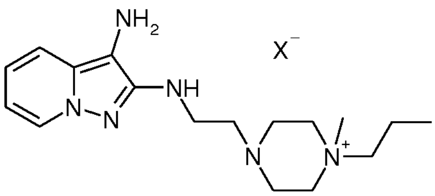 4-[2-(3-Aminopyrazolo[1,5-a]pyridin-2-ylamino)ethyl]-1-methyl-1-propylpiperazin-1-ium salt |
| 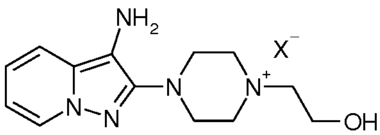 4-(3-Aminopyrazolo[1,5-a]pyridin-2-yl)-1-(2-hydroxyethyl)piperazin-1-ium salt |
| 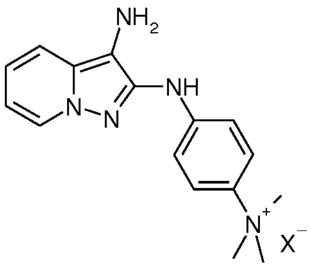 [4-(3-Aminopyrazolo[1,5-a]pyridin-2-ylamino)phenyl]trimethylammonium salt |

(continued)

| |
|---|
| 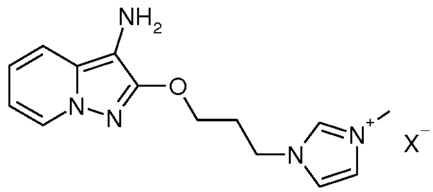 3-[3-(3-Aminopyrazolo[1,5-a]pyridin-2-yloxy)propyl]-1-methyl-3H-imidazol-1-ium salt |
| 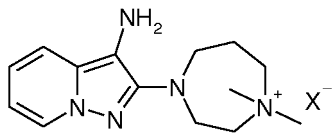 4-(3-Aminopyrazolo[1,5-a]pyridin-2-yl)-1,1-dimethyl-1,4-diazepan-1-ium salt |
| 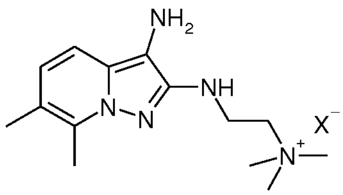 [2-(3-Amino-6,7-dimethylpyrazolo[1,5-a]pyridin-2-ylamino)ethyl]trimethylammonium salt |
| 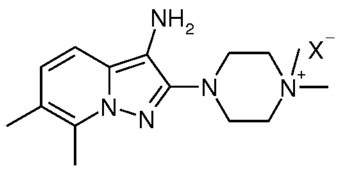 4-(3-Amino-6,7-dimethylpyrazolo[1,5-a]pyridin-2-yl)-1,1-dimethylpiperazin-1-ium salt |
| 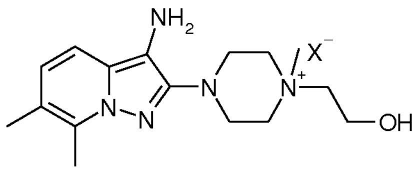 4-(3-Amino-6,7-dimethylpyrazolo[1,5-a]pyridin-2-yl)-1-(2-hydroxyethyl)-1-methylpiperazin-1-ium salt |
| 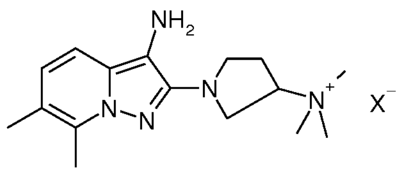 [1-(3-Amino-6,7-dimethylpyrazolo[1,5-a]pyridin-2-yl)pyrrolidin-3-yl]trimethylammonium salt |
| 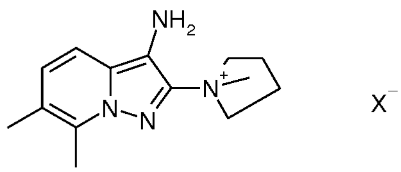 1-(3-Amino-6,7-dimethylpyrazolo[1,5-a]pyridin-2-yl)-1-methylpyrrolidinium salt |

(continued)

| |
|---|
| 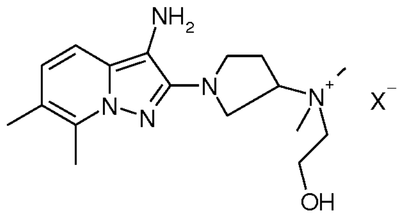 [1-(3-Amino-6,7-dimethylpyrazolo[1,5-a]pyridin-2-yl)pyrrolidin-3-yl](2-hydroxyethyl)dimethylammonium salt |
| 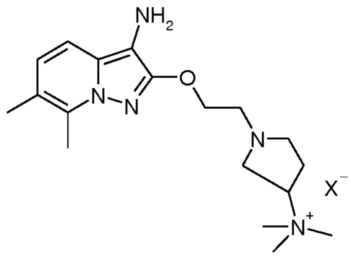 {1-[2-(3-Amino-6,7-dimethylpyrazolo[1,5-a]pyridin-2-yloxy)ethyl]pyrrolidin-3-yl}trimethylammonium salt |
| 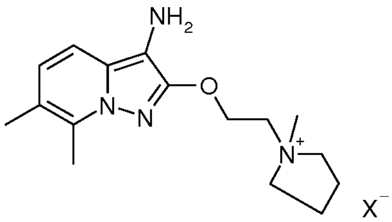 1-{2-[(3-Amino-6,7-dimethylpyrazolo[1,5-a]pyridin-2-yl)oxy]ethyl}-1-methylpyrrolidinium salt |
| 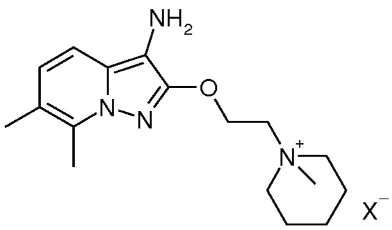 1-{2-[(3-Amino-6,7-dimethylpyrazolo[1,5-a]pyridin-2-yl)oxy]ethyl}-1-methylpiperidinium salt |
| 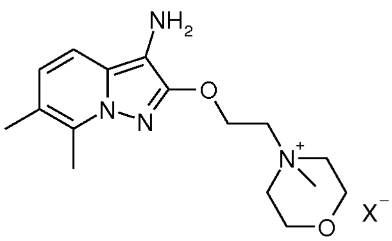 4-{2-[(3-Amino-6,7-dimethylpyrazolo[1,5-a]pyridin-2-yl)oxy]ethyl}-4-methylmorpholin-4-ium salt |
| 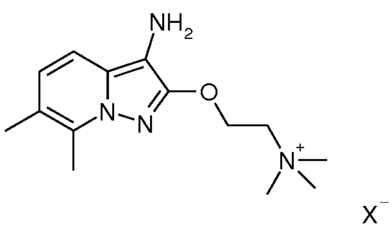 {2-[(3-Amino-6,7-dimethylpyrazolo[1,5-a]pyridin-2-yl)oxy]ethyl}trimethylammonium salt |

(continued)

| |
|---|
| 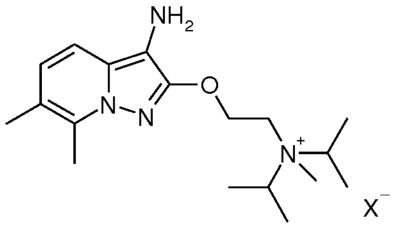<br>{2-[(3-Amino-6,7-dimethylpyrazolo[1,5-a]pyridin-2-yl)oxy]ethyl}diisopropylmethylammonium salt |
| 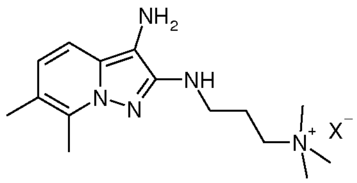<br>[3-(3-Amino-6,7-dimethylpyrazolo[1,5-a]pyridin-2-ylamino)propyl]trimethylammonium salt |
| 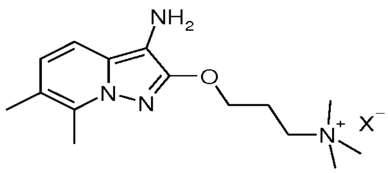<br>[3-(3-Amino-6,7-dimethylpyrazolo[1,5-a]pyridin-2-yloxy)propyl]trimethylammonium salt |

3. Composition according to any one of the preceding claims, **characterized in that** the content of compound of formula (I) is between 0.001 and 10% by weight approximately of the total weight of the dyeing composition.

4. Composition according to any one of the preceding claims, **characterized in that** the coupler or couplers are chosen from meta-phenylenediamines, meta-aminophenols, meta-diphenols, naphthalene couplers, heterocyclic couplers and their addition salts, the amount of each of the couplers being between 0.001 and 10% by weight approximately of the total weight of the dyeing composition.

5. Composition according to any one of the preceding claims, in which the alkyl ether carboxylic acid or acids and their salts are chosen from the compounds of following formula (II):

$$R_8 - A - B - [CH_2]_{q'} - COOX' \quad (II)$$

in which:

$R_8$ represents a linear or branched $C_8$-$C_{22}$ alkyl or alkylene radical or a ($C_8$-$C_9$)alkylaryl radical, such as a ($C_8$-$C_9$)alkylphenyl radical;
A represents an oxygen atom or a -CO-, -NH- or -CO-O-group;
B is composed of a random or block sequence of p' units $-C_3H_6O-$ and of n' units $-C_2H_4O-$;
n' is an integer ranging from 1 to 30;
p' is an integer ranging from 0 to 15;
q' is an integer equal to 0 or 1;
X' denotes a hydrogen atom or else Na, K, Li, ½ Mg or a monoethanolamine, ammonium or triethanolamine residue.

6. Composition according to the preceding claim, **characterized in that**, in the formula (II), $R_8$ denotes a $C_8$-$C_{22}$, preferably $C_{10}$-$C_{18}$, alkyl radical, A represents an oxygen atom, X' denotes a hydrogen or sodium atom, p' = 0, n' varies from 1 to 20, and preferably from 1 to 10, and q' is equal to 0 or 1.

**7.** Composition according to any one of the preceding claims, in which the alkyl polyglucoside or polyglucosides are represented by the following general formula:

$$R_9O\text{-}(R_{10}O)_{t'}\text{-}(G)_v \qquad (III)$$

in which $R_9$ represents a linear or branched alkyl and/or alkylene radical comprising approximately from 12 to 30 carbon atoms or an alkylphenyl radical, the linear or branched alkyl radical of which comprises from 12 to 30 carbon atoms, $R_{10}$ represents one or more alkylene radicals comprising approximately from 2 to 4 carbon atoms, G represents a sugar unit comprising from 5 to 6 carbon atoms, t' denotes a value ranging from 0 to 10, preferably from 0 to 4, and v denotes a value ranging from 1 to 15.

**8.** Composition according to any one of the preceding claims, in which the monoglycerolated or polyglycerolated surface-active agent or agents are preferably chosen from the compounds with the following formulae: $R_{11}O[CH_2CH(CH_2OH)O]_mH$, $R_{11}O[CH_2CH(OH)CH_2O]_mH$ or $R_{11}O[CH(CH_2OH)CH_2O]_mH$, in which $R_{11}$ represents a saturated or unsaturated and linear or branched hydrocarbon radical comprising from 8 to 40 carbon atoms and preferably from 10 to 30 carbon atoms and m is a number between 1 and 30.

**9.** Composition according to Claim 8, **characterized in that** $R_{11}$ comprises heteroatoms.

**10.** Composition according to Claim 8 or 9, **characterized in that** $R_{11}$ denotes a $C_{10}$-$C_{20}$ alkyl or alkylene radical which is optionally mono- or polyhydroxylated.

**11.** Composition according to any one of the preceding claims, **characterized in that** the surface-active agent or agents are chosen from lauryl ether carboxylic acid, ($C_{12}$/$C_{14}$/$C_{16}$ 68/26/6) alkyl polyglucoside and polyglycerolated cetearyl alcohol comprising r mol of glycerol, r being an integer between 2 and 10.

**12.** Composition according to any one of the preceding claims, **characterized in that** the surface-active agent or agents are each present in an amount of between 0.01% and 30% by weight of the total weight of the dyeing composition.

**13.** Method for the oxidation dyeing of keratinous fibres, **characterized in that** a dyeing composition as defined in any one of Claims 1 to 12 is applied to the fibres in the presence of an oxidizing agent for a time sufficient to develop the desired colouring.

**14.** Method according to Claim 13, **characterized in that** the oxidizing agent is chosen from hydrogen peroxide, urea hydrogen peroxide, alkali metal bromates, persalts, peracids and oxidase enzymes.

**15.** Multicompartment device, in which a first compartment comprises a dyeing composition as defined in any one of Claims 1 to 12 and a second compartment comprises an oxidizing agent.

**Patentansprüche**

**1.** Färbezusammensetzung, umfassend in einem für das Färben von Keratinfasern geeigneten Färbemedium:

• eine oder mehrere Aminopyrazolopyridin-Oxidationsbasen der folgenden Formel (I'):

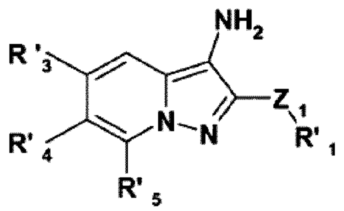

(I')

X

worin:

• $Z_1$ für

- eine kovalente Einfachbindung,
- einen zweiwertigen Rest, ausgewählt aus

  - einem $-O(CH_2)_p$-Rest, wobei p für eine ganze Zahl im Bereich von 0 bis 6 steht,
  - einem $-NR'_6(CH2)_q$-Rest, wobei q für eine ganze Zahl im Bereich von 0 bis 6 steht und $R'_6$ für ein Wasserstoffatom oder einen $C_1$-$C_6$-Alkylrest, der gegebenenfalls durch eine oder mehrere Hydroxygruppen substituiert ist, steht,

steht,

• $R'_1$ für Imidazolgruppen, die durch einen quaternären Ammoniumrest substituiert sind, oder Imidazoliumgruppen, Piperazingruppen, die durch einen quaternären Ammoniumrest substituiert sind, oder Piperaziniumgruppen, Pyrrolidingruppen, die durch einen quaternären Ammoniumrest substituiert sind, oder Pyrrolidiniumgruppen oder Diazepangruppen, die durch einen quaternären Ammoniumrest substituiert sind, oder Diazepaniumgruppen; eine Trialkylammonium-, Tri(hydroxyalkyl)-ammonium-, (Hydroxyalkyl)dialkylammonium- oder Di(hydroxyalkyl)alkylammoniumgruppe steht; $R'_3$, $R'_4$ und $R'_5$ gleich oder verschieden sind und für:

- ein Wasserstoffatom,
- einen gegebenenfalls substituierten $C_1$-$C_4$-Alkylrest

stehen,
• X für ein Ion oder eine Gruppe von Ionen steht, die es möglich macht, die Elektronegativität des Derivats der Formel (I') bereitzustellen,

• einen oder mehrere Kuppler, und
• ein oder mehrere Tenside, die aus ($C_8$-$C_{30}$)-Alkylethercarbonsäuren und deren Salzen, ($C_{12}$-$C_{30}$)-Alkylpolyglucosiden und mono- oder polyglycerinierten oberflächenaktiven Mitteln ausgewählt sind.

**2.** Zusammensetzung nach dem Anspruch 1, worin die Verbindung der Formel (I) ausgewählt ist aus:

| |
|---|
| 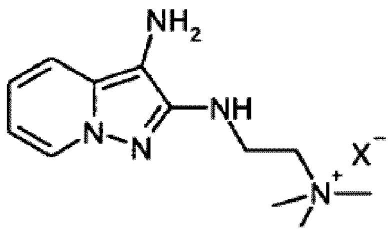<br>[2-(3-Aminopyrazolo[1,5-a]pyridin-2-ylamino)ethyl]trimethylammoniumsalz |
| 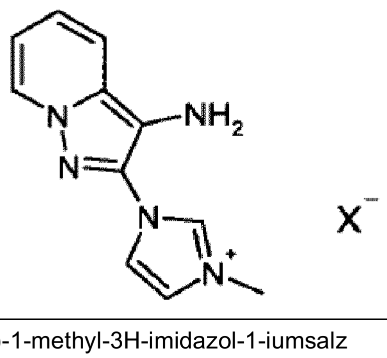 |
| 3-(3-Aminopyrazolo[1,5-a]pyridin-2-yl)-1-methyl-3H-imidazol-1-iumsalz |

(fortgesetzt)

| |
|---|
| 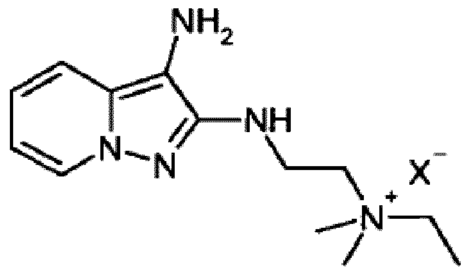<br>[2-(3-Aminopyrazolo[1,5-a]pyridin-2-ylamino)ethyl]ethyldimethylammoniumsalz |
| 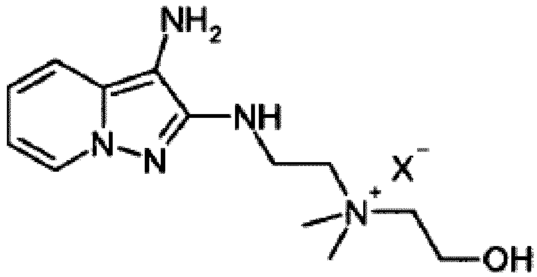<br>[2-(3-Aminopyrazolo[1,5-a]pyridin-2-ylamino)ethyl](2-hydroxyethyl)dimethylammoniumsalz |
| 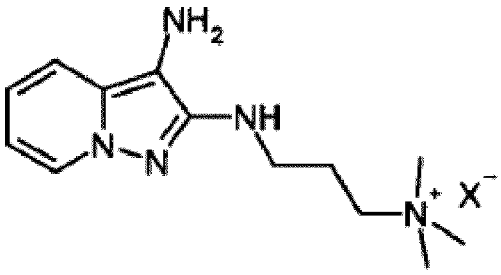<br>[3-(3-Aminopyrazolo[1,5-a]pyridin-2-ylamino)propyl]trimethylammoniumsalz |
| 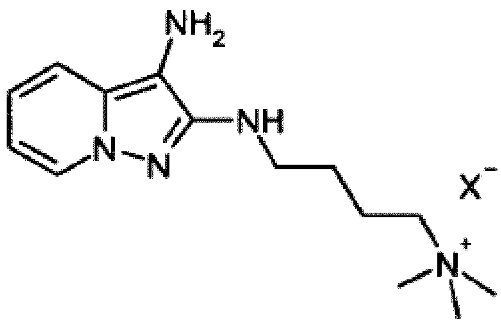<br>[4-(3-Aminopyrazolo[1,5-a]pyridin-2-ylamino)butyl]-trimethylammoniumsalz |
| 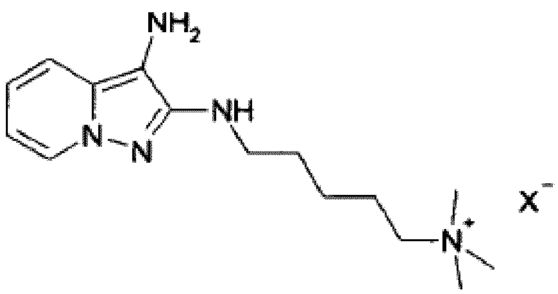<br>[5-(3-Aminopyrazolo[1,5-a]pyridin-2-ylamino)pentyl]trimethylammoniumsalz |

(fortgesetzt)

| |
|---|
| 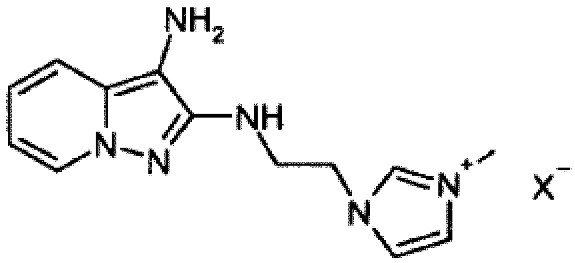<br>3-[2-(3-Aminopyrazolo[1,5-a]pyridin-2-ylamino)ethyl]-1-methyl-3H-imidazol-1-iumsalz |
| 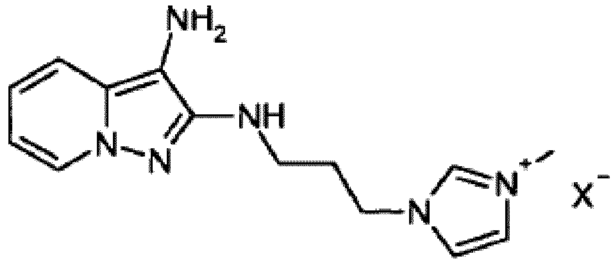<br>3-[3-(3-Aminopyrazolo[1,5-a]pyridin-2-ylamino)propyl]-1-methyl-3H-imidazol-1-iumsalz |
| 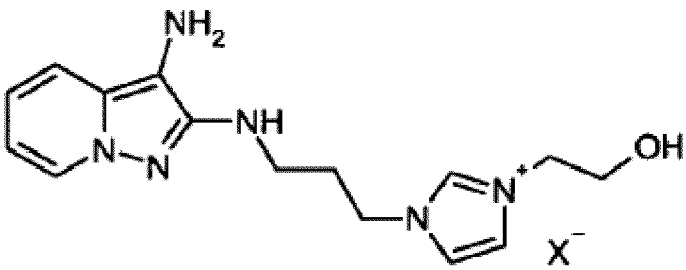<br>3-[3-(3-Aminopyrazolo[1,5-a]pyridin-2-ylamino)propyl]-1-(2-hydroxyethyl)-3H-imidazol-1-iumsalz |
| 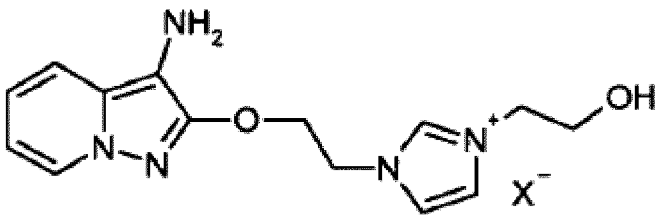<br>3-[2-(3-Aminopyrazolo[1,5-a]pyridin-2-yloxy)ethyl]-1-(2-hydroxyethyl)-3H-imidazol-1-iumsalz |
| 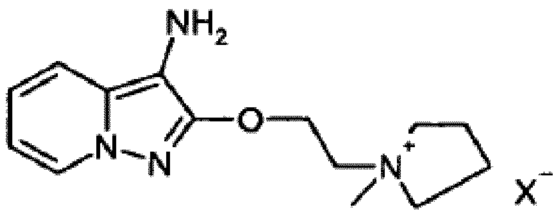<br>1-{2-[(3-Aminopyrazolo[1,5-a]pyridin-2-yl)oxy]ethyl}-1-methylpyrrolidiniumsalz |
| 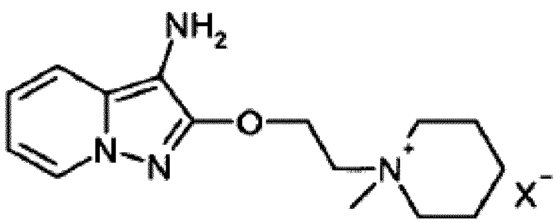 |

(fortgesetzt)

| |
|---|
| 1-{2-[(3-Aminopyrazolo[1,5-a]pyridin-2-yl)oxy]ethyl}-1-methylpiperidiniumsalz |
| 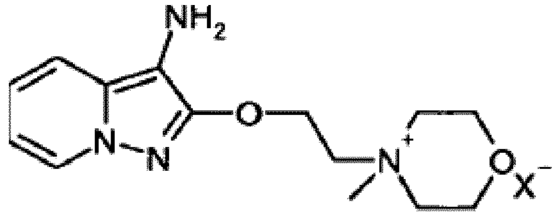 4-{2-[(3-Aminopyrazolo[1,5-a]pyridin-2-yl)oxy]ethyl}-4-methylmorpholin-4-iumsalz |
| 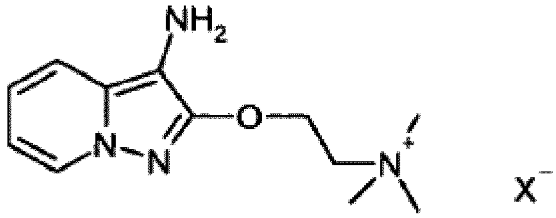 {2-[(3-Aminopyrazolo[1,5-a]pyridin-2-yl)oxy]ethyl}trimethylammoniumsalz |
| 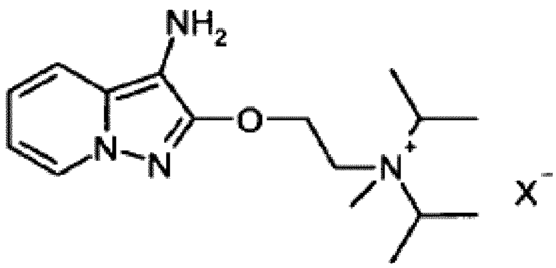 {2-[(3-Aminopyrazolo[1,5-a]pyridin-2-yl)oxy]ethyl}diisopropylmethylammoniumsalz |
| 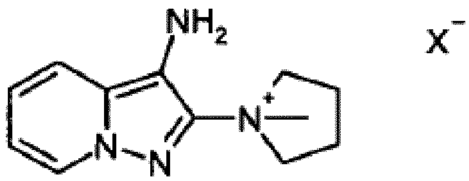 1-(3-Aminopyrazolo[1,5-a]pyridin-2-yl)-1-methylpyrrolidiniumsalz |
| 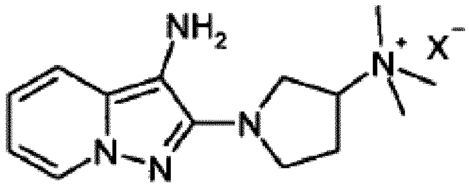 [1-(3-Aminopyrazolo[1,5-a]pyridin-2-yl)pyrrolidin-3-yl]trimethylammoniumsalz |
| 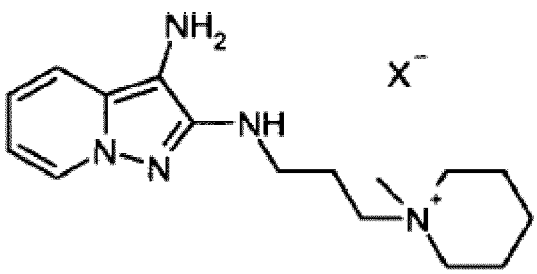 1-[3-(3-Aminopyrazolo[1,5-a]pyridin-2-ylamino)propyl]-1-methylpiperidiniumsalz |

(fortgesetzt)

| |
|---|
| 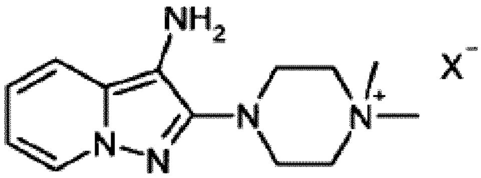<br>4-(3-Aminopyrazolo[1,5-a]pyridin-2-yl)-1,1-dimethylpiperazin-1-iumsalz |
| 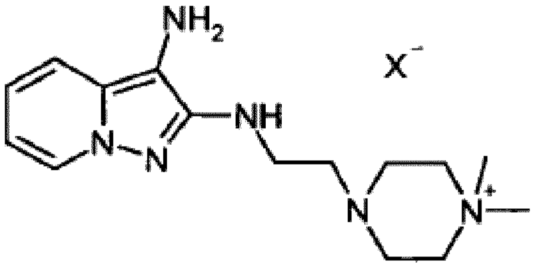<br>4-[2-(3-Aminopyrazolo[1,5-a]pyridin-2-ylamino)ethyl]-1,1-dimethylpiperazin-1-iumsalz |
| 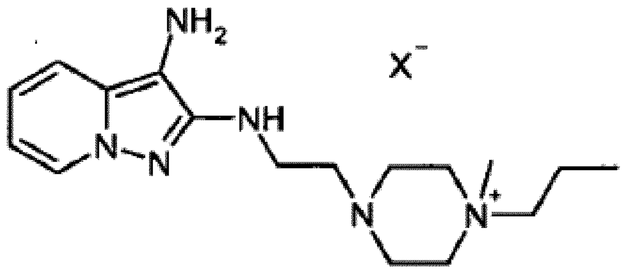<br>4-[2-(3-Aminopyrazolo[1,5-a]pyridin-2-ylamino)ethyl]-1-methyl-1-propylpiperazin-1-iumsalz |
| 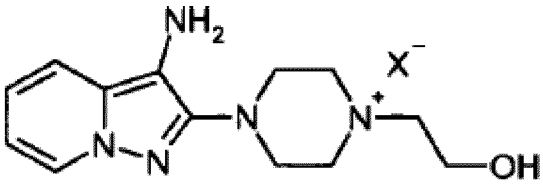<br>4-(3-Aminopyrazolo[1,5-a]pyridin-2-yl)-1-(2-hydroxyethyl)piperazin-1-iumsalz |
| 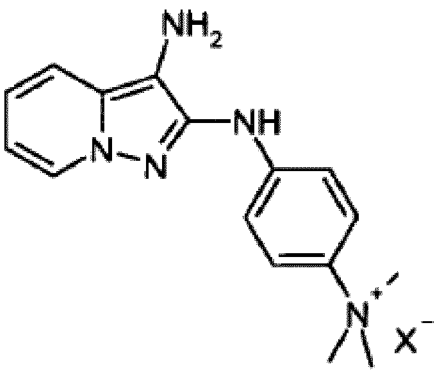<br>[4-(3-Aminopyrazolo[1,5-a]pyridin-2-ylamino)phenyl]trimethylammoniumsalz |

(fortgesetzt)

| |
|---|
| 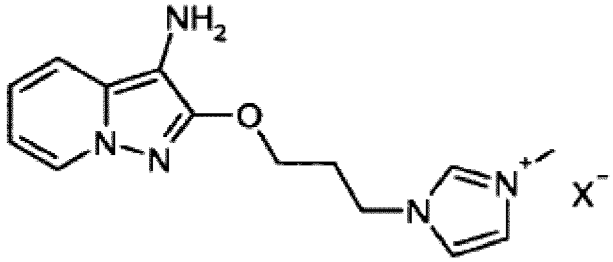 3-[3-(3-Aminopyrazolo[1,5-a]pyridin-2-yloxy)propyl]-1-methyl-3H-imidazol-1-iumsalz |
| 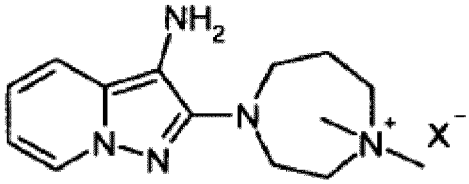 4-(3-Aminopyrazolo[1,5-a]pyridin-2-yl)-1,1-dimethyl-[1,4]diazepan-1-iumsalz |
| 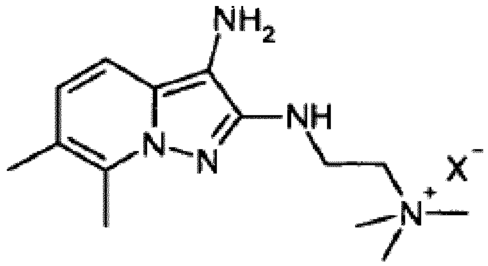 [2-(3-Amino-6,7-dimethylpyrazolo[1,5-a]pyridin-2-ylamino)ethyl]trimethylammoniumsalz |
| 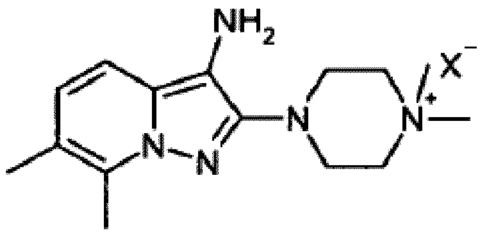 4-(3-Amino-6,7-dimethylpyrazolo[1,5-a]pyridin-2-yl)-1,1-dimethylpiperazin-1-iumsalz |
| 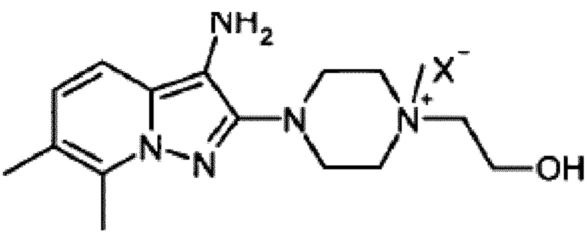 4-(3-Amino-6,7-dimethylpyrazolo[1,5-a]pyridin-2-yl)-1-(2-hydroxyethyl)-1-methylpiperazin-1-iumsalz |
| 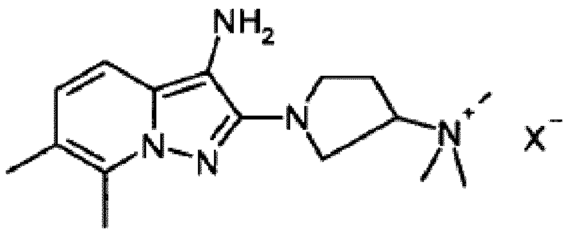 [1-(3-Amino-6,7-dimethylpyrazolo[1,5-a]pyridin-2-yl)pyrrolidin-3-yl]trimethylammoniumsalz |

(fortgesetzt)

| |
|---|
| 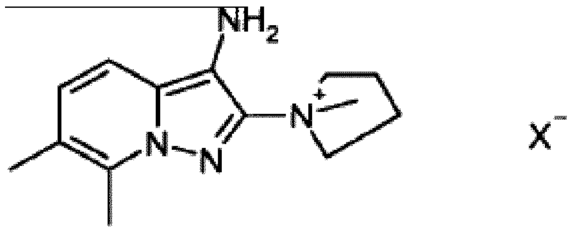<br>1-(3-Amino-6,7-dimethylpyrazolo[1,5-a]pyridin-2-yl)-1-methylpyrrolidiniumsalz |
| 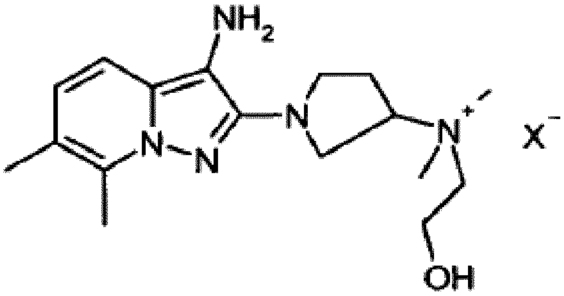<br>[1-(3-Amino-6,7-dimethylpyrazolo[1,5-a]pyridin-2-yl)pyrrolidin-3-yl](2-hydroxyethyl)dimethylammoniumsalz |
| 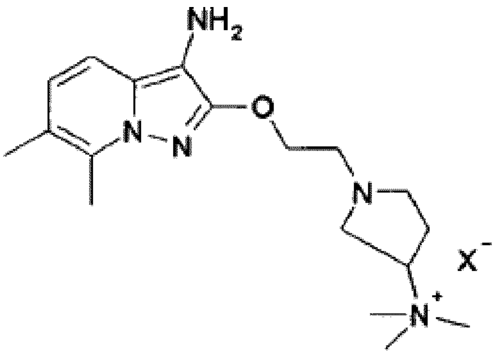<br>{1-[2-(3-Amino-6,7-dimethylpyrazolo[1,5-a]pyridin-2-yloxy)ethyl]pyrrolidin-3-yl}trimethylammoniumsalz |
| 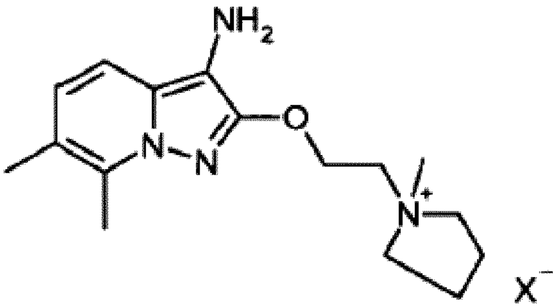<br>1-{2-[(3-Amino-6,7-dimethylpyrazolo[1,5-a]pyridin-2-yl)oxy]ethyl}-1-methylpyrrolidiniumsalz |
| 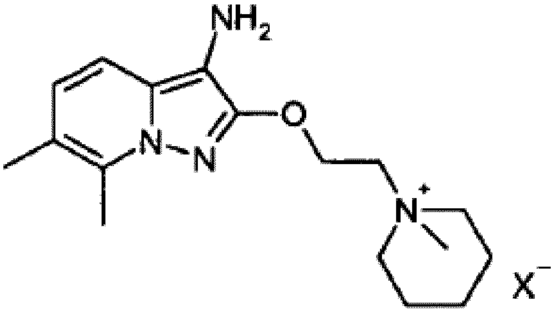 |

(fortgesetzt)

| |
|---|
| 1-{2-[(3-Amino-6,7-dimethylpyrazolo[1,5-a]pyridin-2-yl)oxy]ethyl}-1-methylpiperidiniumsalz |
| 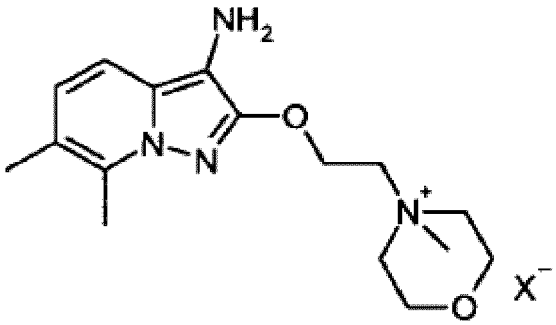 4-{2-[(3-Amino-6,7-dimethylpyrazolo[1,5-a]pyridin-2-yl)oxy]ethyl}-4-methylmorpholin-4-iumsalz |
| 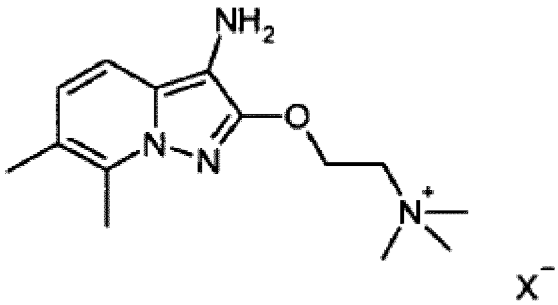 {2-[(3-Amino-6,7-dimethylpyrazolo[1,5-a]pyridin-2-yl)oxy]ethyl}trimethylammoniumsalz |
| 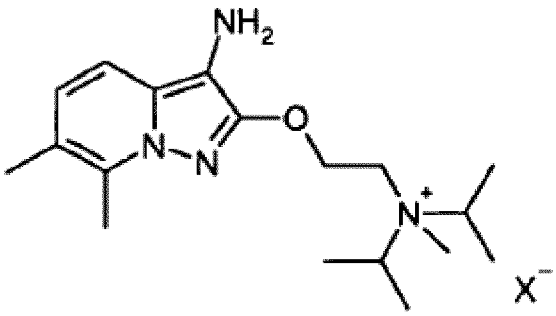 {2-[(3-Amino-6,7-dimethylpyrazolo[1,5-a]pyridin-2-yl)oxy]ethyl}diisopropylmethylammoniumsalz |
| 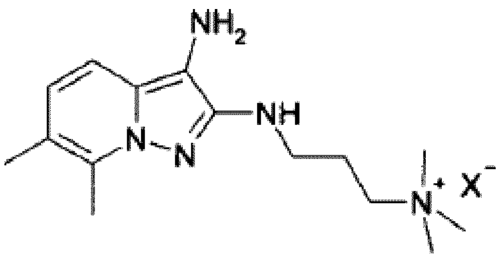 [3-(3-Amino-6,7-dimethylpyrazolo[1,5-a]pyridin-2-ylamino)propyl]trimethylammoniumsalz |
| 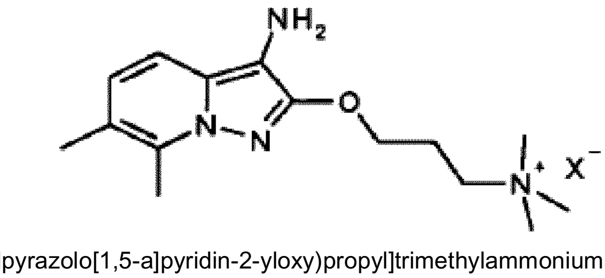 [3-(3-Amino-6,7-dimethylpyrazolo[1,5-a]pyridin-2-yloxy)propyl]trimethylammoniumsalz |

**3.** Zusammensetzung nach einem der vorhergehenden Ansprüche, **dadurch gekennzeichnet, dass** der Gehalt an der Verbindung der Formel (I) zwischen etwa 0,001 und 10 Gew.-%, bezogen auf das Gesamtgewicht der Färbezusammensetzung, liegt.

**4.** Zusammensetzung nach einem der vorhergehenden Ansprüche, **dadurch gekennzeichnet, dass** der Kuppler bzw. die Kuppler aus meta-Phenylendiaminen, meta-Aminophenolen, meta-Diphenolen, Naphthalin-Kupplern, heterocyclischen Kupplern und Additionssalzen davon ausgewählt ist bzw. sind, wobei die Menge jedes der Kuppler zwischen etwa 0,001 und 10 Gew.-%, bezogen auf das Gesamtgewicht der Färbezusammensetzung, liegt.

**5.** Zusammensetzung nach einem der vorhergehenden Ansprüche, wobei die Alkyethercarbonsäure bzw. Alkyethercarbonsäuren und deren Salze aus den Verbindungen der folgenden Formel (II) ausgewählt ist bzw. sind:

$$R_8 - A - B - \left[ CH_2 \right]_{q'} - COOX' \qquad (II)$$

worin:

$R_8$ für einen linearen oder verzweigten $C_8$-$C_{22}$-Alkyl- oder -Alkylenrest oder einen ($C_8$-$C_9$)-Alkylarylrest, wie einen ($C_8$-$C_9$)-Alkylphenylrest, steht;
A für ein Sauerstoffatom oder eine -CO-, -NH- oder -CO-O-Gruppe steht;
B aus einer statistischen oder blockartigen Sequenz von p' Einheiten -$C_3H_6O$- und n' Einheiten - $C_2H_4O$- aufgebaut ist;
n' für eine ganze Zahl im Bereich von 1 bis 30 steht;
p' für eine ganze Zahl im Bereich von 0 bis 15 steht;
q' für eine ganze Zahl mit einem Wert von 0 oder 1 steht;
X' für ein Wasserstoffatom oder auch Na, K, Li, ½ Mg oder einen Monoethanolamin-, Ammonium- oder Triethanolaminrest steht.

**6.** Zusammensetzung nach dem vorhergehenden Anspruch, **dadurch gekennzeichnet, dass** in der Formel (II) $R_8$ für einen $C_8$-$C_{22}$-Alkylrest, vorzugsweise $C_{10}$-$C_{18}$-Alkylrest, steht, A für ein Sauerstoffatom steht, X' für ein Wasserstoff- oder Natriumatom steht, p' = 0, n' von 1 bis 20 und vorzugsweise von 1 bis 10 variiert und q' gleich 0 oder 1 ist.

**7.** Zusammensetzung nach einem der vorhergehenden Ansprüche, wobei das Alkylpolyglucosid bzw. die Alkylpolyglucoside durch die folgende allgemeine Formel wiedergegeben wird bzw. werden:

$$R_9O\text{-}(R_{10}O)_{t'}\ (G)_v \qquad (III)$$

worin $R_9$ für einen linearen oder verzweigten Alkyl- und/oder Alkenylrest mit etwa 12 bis 30 Kohlenstoffatomen oder einen Alkylphenylrest, dessen linearer oder verzweigter Alkylrest 12 bis 30 Kohlenstoffatome enthält, steht, $R_{10}$ für einen oder mehrere Alkylenreste mit etwa 2 bis 4 Kohlenstoffatomen steht, G für eine Zuckereinheit mit 5 bis 6 Kohlenstoffatomen steht, t' für einen Wert im Bereich von 0 bis 10, vorzugsweise von 0 bis 4, steht und v für einen Wert im Bereich von 1 bis 15 steht.

**8.** Zusammensetzung nach einem der vorhergehenden Ansprüche, wobei das mono- oder polyglycerinierte oberflächenaktive Mittel bzw. die mono- oder polyglycerinierten oberflächenaktiven Mittel aus den Verbindungen mit den folgenden Formeln ausgewählt sind: $R_{11}O[CH_2CH(CH_2OH)O]_mH$, $R_{11}O[CH_2CH(OH)CH_2O]_mH$ oder $R_{11}O[CH(CH_2OH)CH_2O]_mH$, wobei $R_{11}$ für einen gesättigten oder ungesättigten und linearen oder verzweigten Kohlenwasserstoffrest mit 8 bis 40 Kohlenstoffatomen und vorzugsweise 10 bis 30 Kohlenstoffatomen steht und m für eine Zahl zwischen 1 und 30 steht.

**9.** Zusammensetzung nach Anspruch 8, **dadurch gekennzeichnet, dass** $R_{11}$ Heteroatome umfasst.

**10.** Zusammensetzung nach Anspruch 8 oder 9, **dadurch gekennzeichnet, dass** $R_{11}$ für einen $C_{10}$-$C_{20}$-Alkyl- oder -Alkylenrest, der gegebenenfalls mono- oder polyhydroxyliert ist, steht.

**11.** Zusammensetzung nach einem der vorhergehenden Ansprüche, **dadurch gekennzeichnet, dass** das oberflächenaktive Mittel bzw. die oberflächenaktiven Mittel aus Laurylethercarbonsäure, ($C_{12}/C_{14}/C_{16}$ 68/26/6)-Alkylpolyglucosid und polyglyceriniertem Cetearylalkohol mit r mol Glycerin, wobei r für eine ganze Zahl zwischen 2 und 10 steht, ausgewählt ist bzw. sind.

**12.** Zusammensetzung nach einem der vorhergehenden Ansprüche, **dadurch gekennzeichnet, dass** das oberflächenaktive Mittel bzw. die oberflächenaktiven Mittel jeweils in einer Menge zwischen 0,01 und 30 Gew.-%, bezogen auf das Gesamtgewicht der Färbezusammensetzung, vorliegen.

**13.** Verfahren zum Oxidationsfärben von Keratinfasern, **dadurch gekennzeichnet, dass** man auf die Fasern eine Färbezusammensetzung gemäß einem der Ansprüche 1 bis 12 in Gegenwart eines Oxidationsmittels über einen zur Entwicklung der gewünschten Färbung ausreichenden Zeitraum aufbringt.

**14.** Verfahren nach Anspruch 13, bei dem man das Oxidationsmittel aus Wasserstoffperoxid, Harnstoffperoxid, Alkalimetallbromaten, Persalzen, Persäuren und Oxidaseenzymen auswählt.

**15.** Vorrichtung mit mehreren Kompartimenten, in der ein erstes Kompartiment eine Färbezusammensetzung gemäß einem der Ansprüche 1 bis 12 enthält und ein zweites Kompartiment ein Oxidationsmittel enthält.

## Revendications

**1.** Composition tinctoriale comprenant, dans un milieu de teinture approprié pour la teinture des fibres kératiniques,

• une ou plusieurs bases d'oxydation aminopyrazolopyridine de formule (I') suivante :

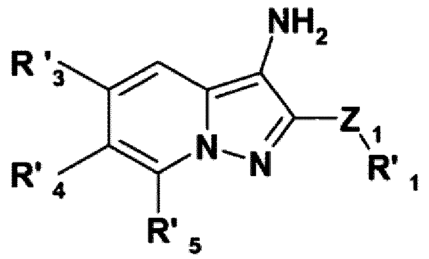

(I')

X

dans laquelle

• $Z_1$ représente indépendamment

- une liaison covalente simple,
- un radical divalent choisi parmi

  - un radical $-O(CH_2)_p-$, p désignant un nombre entier allant de 0 à 6,
  - un radical $-NR'_6(CH_2)_q(C_6H_4)_t-$, q désignant un nombre entier allant de 0 à 6 et $R'_6$ représentant un atome d'hydrogène ou un radical alkyle en C1-C6 éventuellement substitué par un ou plusieurs groupements hydroxy,
  - $R'_1$ représente des imidazoles substitués par un radical ammonium quaternaire ou des imidazoliums, des pipérazines substitués par un radical ammonium quaternaire ou des pipéraziniums, des pyrrolidines substitués par un radical ammonium quaternaire ou des pyrrolidiniums, ou des diazépanes substitués par un radical ammonium quaternaire ou des diazépaniums ; un groupe trialkylammonium, tri(hydroxyalkyl)ammonium, (hydroxyalkyl)dialkylammonium ou di(hydroxyalkyl)alkylammonium ;

• $R'_3$, $R'_4$ et $R'_5$, identiques ou différents, représentent

- un atome d'hydrogène,
- un radical alkyle en $C_1$-$C_4$ éventuellement substitué,

• X représente un ion ou groupe d'ions permettant d'assurer l'électronégativité du dérivé de formule (I'),

• un ou plusieurs coupleurs, et
• un ou plusieurs tensioactifs choisis parmi les acides alkyl ($C_8$-$C_{30}$) éther carboxyliques et leurs sels, les alkyl ($C_{12}$-$C_{30}$) polyglucosides et les agents tensioactifs mono ou polyglycérolés.

**2.** Composition selon la revendication 1 dans laquelle le composé de formule (I) est choisi parmi :

| |
|---|
| 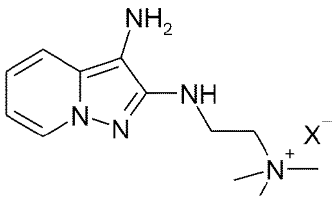 Sel de [2-(3-amino-pyrazolo[1,5-a]pyridin-2-ylamino)-ethyl]-trimethyl-ammonium |
| 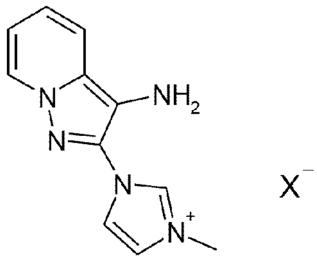 Sel de 3-(3-amino-pyrazolo[1,5-a]pyridin-2-yl)-1-methyl-3H-imidazol-1-ium |
| 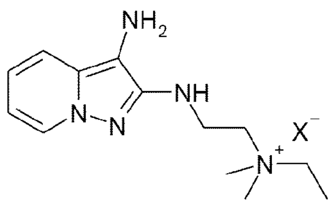 Sel de [2-(3-amino-pyrazolo[1,5-a]pyridin-2-ylamino)-ethyl]-ethyl-dimethyl-ammonium |
| 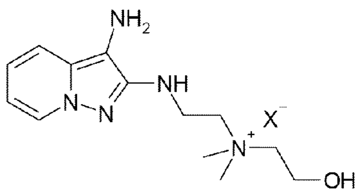 Sel de [2-(3-amino-pyrazolo[1,5-a]pyridin-2-ylamino)-ethyl]-(2-hydroxy-ethyl)-dimethyl-ammonium |
| 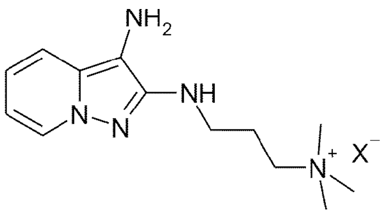 Sel de [3-(3-amino-pyrazolo[1,5-a]pyridin-2-ylamino)-propyl]-trimethyl-ammonium |

(suite)

| |
|---|
| 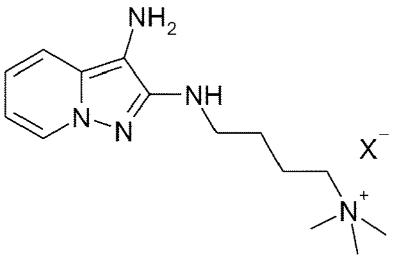 Sel de [4-(3-amino-pyrazolo[1,5-a]pyridin-2-ylamino)-butyl]-trimethyl-ammonium |
| 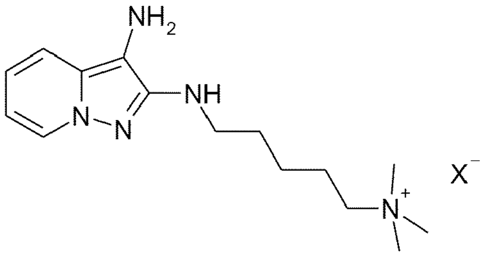 Sel de [5-(3-amino-pyrazolo[1,5-a]pyridin-2-ylamino)-pentyl]-trimethyl-ammonium |
| 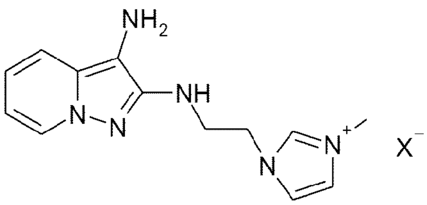 Sel de 3-[2-(3-amino-pyrazolo[1,5-a]pyridin-2-ylamino)-ethyl]-1-methyl-3H-imidazol-1-ium |
| 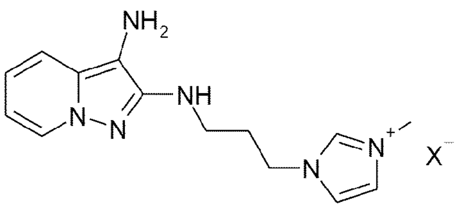 Sel de 3-[3-(3-amino-pyrazolo[1,5-a]pyridin-2-ylamino)-propyl]-1-methyl-3H-imidazol-1-ium |
| 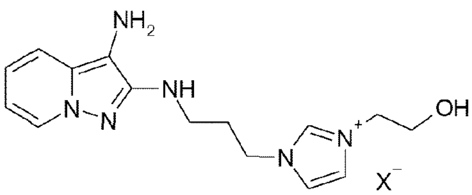 Sel de 3-[3-(3-amino-pyrazolo[1,5-a]pyridin-2-ylamino)-propyl]-1-(2-hydroxy-ethyl)-3H-imidazol-1-ium |
| 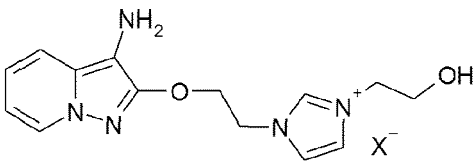 Sel de 3-[2-(3-amino-pyrazolo[1,5-a]pyridin-2-yloxy)-ethyl]-1-(2-hydroxy-ethyl)-3H-imidazol-1-ium |

(suite)

| |
|---|
| 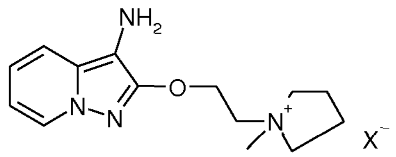<br>Sel de 1-{2-[(3-amino-pyrazolo[1,5-a]pyridin-2-yl)oxy]ethyl}-1-methylpyrrolidinium |
| 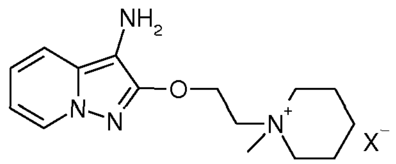<br>Sel de 1-{2-[(3-amino-pyrazolo[1,5-a]pyridin-2-yl)oxy]ethyl}-1-methylpiperidinium |
| 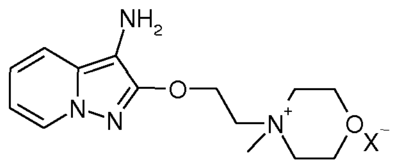<br>Sel de 4-{2-[(3-amino-pyrazolo[1,5-a]pyridin-2-yl)oxy]ethyl}-4-methylmorpholin-4-ium |
| 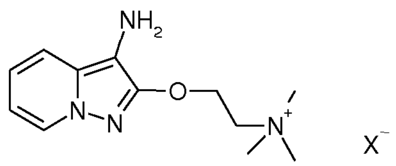<br>Sel de {2-[(3-amino-pyrazolo[1,5-a]pyridin-2-yl)oxy]ethyl}-trimethyl-ammonium |
| 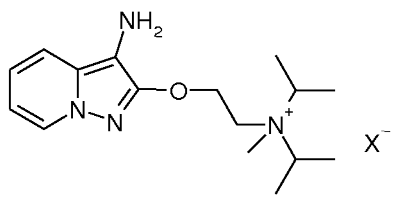<br>Sel de {2-[(3-amino-pyrazolo[1,5-a]pyridin-2-yl)oxy]ethyl}-diisopropyl-methyl-ammonium |
| 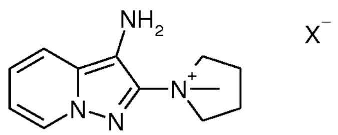<br>Sel de 1-(3-amino-pyrazolo[1,5-a]pyridin-2-yl)-1-methylpyrrolidinium |
| 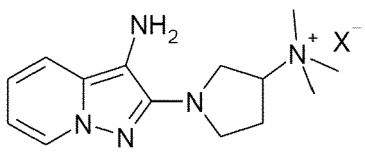<br>Sel de [1-(3-amino-pyrazolo[1,5-a]pyridin-2-yl)-pyrrolidin-3-yl]-trimethyl-ammonium |
| 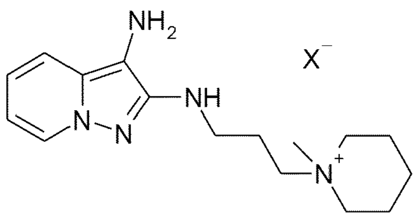 |

(suite)

| |
|---|
| Sel de 1-[3-(3-amino-pyrazolo[1,5-a]pyridin-2-ylamino)-propyl]-1-methylpiperidinium |
| 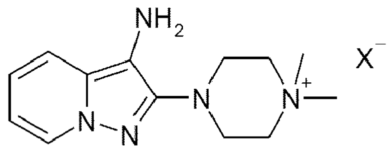 Sel de 4-(3-amino-pyrazolo[1,5-a]pyridin-2-yl)-1,1-dimethyl-piperazin-1-ium |
| 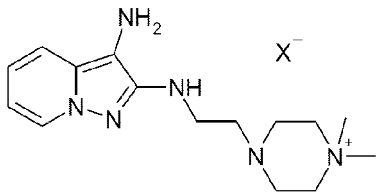 Sel de 4-[2-(3-amino-pyrazolo[1,5-a]pyridin-2-ylamino)-ethyl]-1,1-dimethyl-piperazin-1-ium |
| 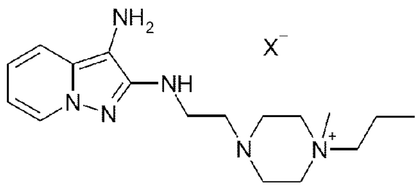 Sel de 4-[2-(3-amino-pyrazolo[1,5-a]pyridin-2-ylamino)-ethyl]-1-methyl-1-propyl-piperazin-1-ium |
| 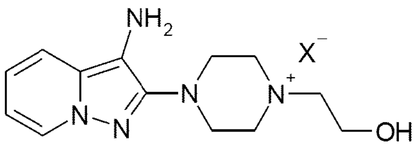 Sel de 4-(3-amino-pyrazolo[1,5-a]pyridin-2-yl)-1-(2-hydroxy-ethyl)-piperazin-1-ium |
| 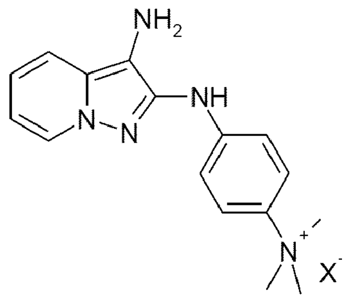 Sel de [4-(3-amino-pyrazolo[1,5-a]pyridin-2-ylamino)-phenyl]-trimethyl-ammonium |
| 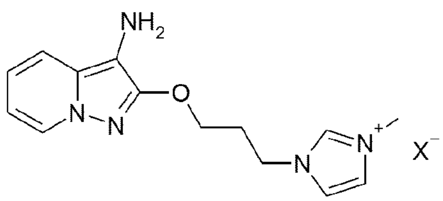 Sel de 3-[3-(3-amino-pyrazolo[1,5-a]pyridin-2-yloxy)-propyl]-1-methyl-3H-imidazol-1-ium |
| 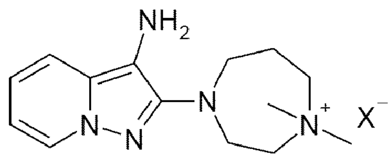 Sel de 4-(3-amino-pyrazolo[1,5-a]pyridin-2-yl)-1,1-dimethyl-[1,4]diazepan-1-ium |

(suite)

| |
|---|
| 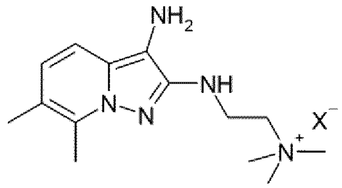<br>Sel de [2-(3-amino-6,7-dimethyl-pyrazolo[1,5-a]pyridin-2-ylamino)-ethyl]-trimethyl-ammonium |
| 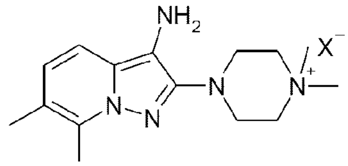<br>Sel de 4-(3-amino-6,7-dimethyl-pyrazolo[1,5-a]pyridin-2-yl)-1,1-dimethyl-piperazin-1-ium |
| 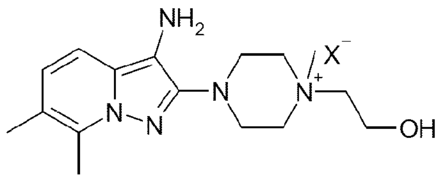<br>Sel de 4-(3-amino-6,7-dimethyl-pyrazolo[1,5-a]pyridin-2-yl)-1-(2-hydroxy-ethyl)-1-methyl-piperazin-1-ium |
| 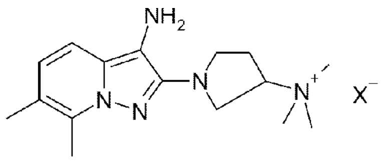<br>Sel de [1-(3-amino-6,7-dimethyl-pyrazolo[1,5-a]pyridin-2-yl)-pyrrolidin-3-yl]-trimethyl-ammonium |
| 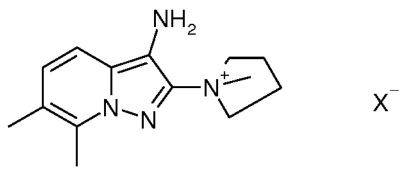<br>Sel de 1-(3-amino-6,7-dimethylpyrazolo[1,5-a]pyridin-2-yl)-1-methylpyrrolidinium |
| 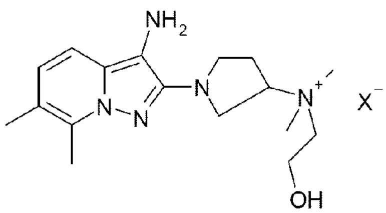<br>Sel de [1-(3-amino-6,7-dimethyl-pyrazolo[1,5-a]pyridin-2-yl)-pyrrolidin-3-yl]-(2-hydroxy-ethyl)-dimethyl-ammonium |
| 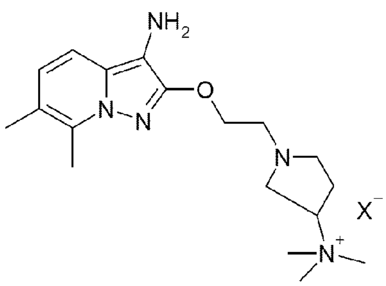 |

(suite)

| |
|---|
| Sel de {1-[2-(3-amino-6,7-dimethyl-pyrazolo[1,5-a]pyridin-2-yloxy)-ethyl]-pyrrolidin-3-yl}-trimethyl-ammonium |
| 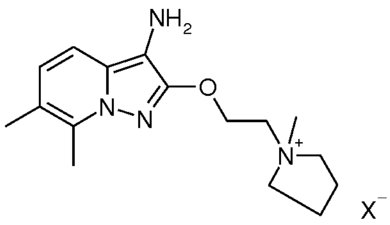 Sel de 1-{2-[(3-amino-6,7-dimethylpyrazolo[1,5-a]pyridin-2-yl)oxy]ethyl}-1-methyl pyrrolidinium |
| 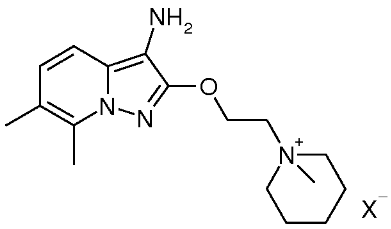 Sel de 1-{2-[(3-amino-6,7-dimethylpyrazolo[1,5-a]pyridin-2-yl)oxy]ethyl}-1-methylpiperidinium |
| 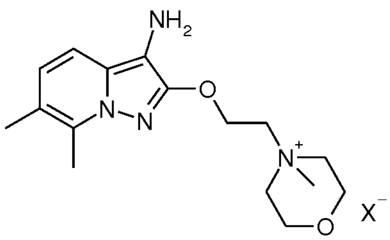 Sel de 4-{2-[(3-amino-6,7-dimethylpyrazolo[1,5-a]pyridin-2-yl)oxy]ethyl}-4-methylmorpholin-4-ium |
| 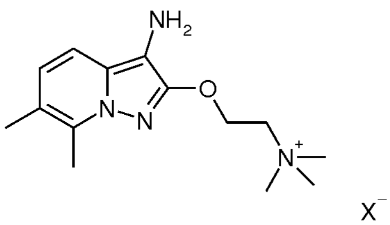 Sel de {2-[(3-amino-6,7-dimethylpyrazolo[1,5-a]pyridin-2-yl)oxy]ethyl}-trimethyl-ammonium |
| 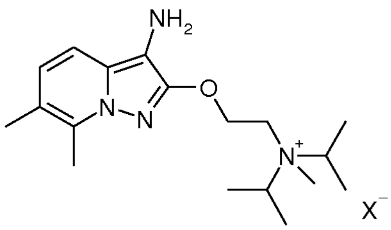 Sel de {2-[(3-amino-6,7-dimethylpyrazolo[1,5-a]pyridin-2-yl)oxy]ethyl}-diisopropyl-methyl-ammonium |
| 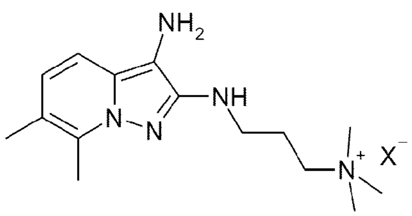 Sel de [3-(3-amino-6,7-dimethyl-pyrazolo[1,5-a]pyridin-2-ylamino)-propyl]-trimethyl-ammonium |

(suite)

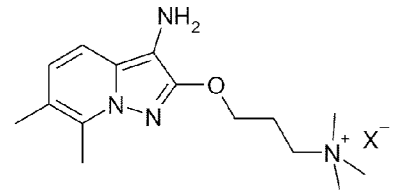

Sel de [3-(3-amino-6,7-dimethyl-pyrazolo[1,5-a]pyridin-2-yloxy)-propyl]-trimethyl-ammonium

**3.** Composition selon l'une quelconque des revendications précédentes, **caractérisée en ce que** la teneur en composé de formule (I) est comprise entre 0,001 à 10 % en poids environ du poids total de la composition tinctoriale.

**4.** Composition selon l'une quelconque des revendications précédentes, **caractérisée en ce que** le ou les coupleurs sont choisis parmi les métaphénylènediamines, les méta-aminophénols, les métadiphénols, les coupleurs naphtaléniques, les coupleurs hétérocycliques et leurs sels d'addition, la quantité de chacun des coupleurs étant comprise entre 0,001 et 10 % en poids environ du poids total de la composition tinctoriale.

**5.** Composition selon l'une quelconque des revendications précédentes dans laquelle le ou les acides alkyl éther carboxyliques et leurs sels sont choisis parmi les composés de formule (II) suivante :

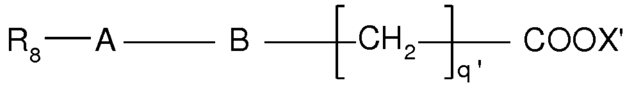

(II)

dans laquelle :

$R_8$ représente un radical alkyle ou alkylène linéaire ou ramifié en $C_8$-$C_{22}$ ou un radical alkyl ($C_8$-$C_9$) aryle, tel qu'un radical alkyl ($C_8$-$C_9$) phényle ;
A représente un atome d'oxygène ou un groupe -CO-, -NH- ou -CO-O- ;
B est constitué d'un enchaînement statistique ou de blocs de p' motifs $-C_3H_6O-$ et de n' motifs $-C_2H_4O-$ ;
n' est un nombre entier allant de 1 à 30 ;
p' est un nombre entier allant de 0 à 15 ;
q' est un nombre entier égal à 0 ou 1 ;
X' désigne un atome d'hydrogène ou bien Na, K, Li, ½ Mg ou un reste monoéthanolamine, ammonium ou triéthanolamine.

**6.** Composition selon la revendication précédente, **caractérisée en ce que**, dans la formule (II), $R_8$ désigne un radical alkyle en $C_8$-$C_{22}$, de préférence en $C_{10}$-$C_{18}$, A représente un atome d'oxygène, X' désigne un atome d'hydrogène ou de sodium, p' = 0, n' varie de 1 à 20, et de préférence de 1 à 10, et q' est égal à 0 ou 1.

**7.** Composition selon l'une quelconque des revendications précédentes dans laquelle le ou les alkyl polyglucosides sont représentés par la formule générale suivante :

$$R_9O\text{-}(R_{10}O)_{t'}\text{-}(G)_v \qquad \text{(III)}$$

dans laquelle $R_9$ représente un radical alkyle et / ou alkylène linéaire ou ramifié comportant environ de 12 à 30 atomes de carbone ou un radical alkylphényle dont le radical alkyle linéaire ou ramifié comporte de 12 à 30 atomes de carbone, $R_{10}$ représente un ou plusieurs radicaux alkylène comportant environ de 2 à 4 atomes de carbone, G représente un motif sucre comportant de 5 à 6 atomes de carbone, t' désigne une valeur allant de 0 à 10, de préférence 0 à 4, et v désigne une valeur allant de 1 à 15.

**8.** Composition selon l'une quelconque des revendications précédentes dans laquelle le ou les agents tensioactifs monoglycérolés ou polyglycérolés sont choisis parmi les composés de formules suivantes : $R_{11}O[CH_2CH(CH_2OH)O]_mH$ ; $R_{11}O[CH_2CH(OH)CH_2O]_mH$ ou $R_{11}O[CH(CH_2OH)CH_2O]_mH$ ; dans lesquelles $R_{11}$ représente un radical hydrocarboné saturé ou insaturé, linéaire ou ramifié, comportant de 8 à 40 atomes de carbone

et de préférence de 10 à 30 atomes de carbone ; m est un nombre compris entre 1 et 30.

**9.** Composition selon la revendication 8, **caractérisée en ce que** $R_{11}$ comprend des hétéroatomes.

**10.** Composition selon la revendication 8 ou 9, **caractérisée en ce que** $R_{11}$ désigne un radical alkyle ou alkylène en $C_{10}$-$C_{20}$, éventuellement mono ou polyhydroxylé.

**11.** Composition selon l'une quelconque des revendications précédentes, **caractérisée en ce que** le ou les agents tensioactifs sont choisis parmi l'acide lauryl éther carboxylique, l'alkyl ($C_{12}$/$C_{14}$/$C_{16}$ 68/26/6) polyglucoside, et l'alcool cétéarylique polyglycérolé à r moles de glycérol, r étant un nombre entier compris entre 2 et 10.

**12.** Composition selon l'une quelconque des revendications précédentes, **caractérisée en ce que** le ou les agents tensioactifs sont chacun présents en quantité comprise entre 0,01 % à 30 % en poids du poids total de la composition tinctoriale.

**13.** Procédé de teinture d'oxydation des fibres kératiniques, **caractérisé en ce qu'**on applique sur les fibres une composition tinctoriale telle que définie dans l'une quelconque des revendications 1 à 12 en présence d'un agent oxydant pendant un temps suffisant pour développer la coloration désirée.

**14.** Procédé selon la revendication 13, **caractérisé en ce que** l'agent oxydant est choisi parmi le peroxyde d'hydrogène, le peroxyde d'urée, les bromates de métaux alcalins, les persels, les peracides et les enzymes oxydases.

**15.** Dispositif à plusieurs compartiments dans lequel un premier compartiment contient une composition tinctoriale telle que définie dans l'une quelconque des revendications 1 à 12 et un deuxième compartiment contient un agent oxydant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2801308
- GB 1026978 A
- GB 1153196 A
- DE 2359399
- JP 63169571 A
- JP 5063124 A
- EP 0770375 A
- WO 9615765 A
- FR 2750048 A
- DE 3843892
- DE 4133957
- WO 9408969 A
- WO 9408970 A
- FR 2733749 A
- DE 19543988
- FR 2886137
- FR 2586913